(19) Europäisches Patentamt / European Patent Office / Office européen des brevets

(11) **EP 4 085 893 B1**

(12) **EUROPÄISCHE PATENTSCHRIFT**


(45) Veröffentlichungstag und Bekanntmachung des Hinweises auf die Patenterteilung:
**26.02.2025 Patentblatt 2025/09**

(21) Anmeldenummer: **21172707.8**

(22) Anmeldetag: **07.05.2021**

(51) Internationale Patentklassifikation (IPC):
***C08F 293/00*** (2006.01) ***C08L 53/00*** (2006.01)
***A61K 6/60*** (2020.01) ***B33Y 70/00*** (2020.01)
***C08F 222/10*** (2006.01) ***A61C 13/087*** (2006.01)
***A61K 6/887*** (2020.01) *B29C 64/124* (2017.01)

(52) Gemeinsame Patentklassifikation (CPC):
(C-Sets verfügbar)
**A61K 6/60; A61C 13/087; B33Y 70/00; C08F 222/10; C08F 293/005; C08L 53/00;**
B29C 64/124; B33Y 10/00; C08F 2438/01 (Forts.)


(54) **TRANSPARENTE, BRUCHZÄHE POLYMERISATIONSHARZE ZUR HERSTELLUNG DENTALER FORMKÖRPER**

TRANSPARENT, FRACTURE-RESISTANT POLYMERISATION RESINS FOR THE PRODUCTION OF DENTAL MOULDINGS

RÉSINES DE POLYMÉRISATION TRANSPARENTES ET RÉSISTANTES À LA RUPTURE DESTINÉES À LA FABRICATION DE CORPS DENTAIRES MOULÉS


(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**09.11.2022 Patentblatt 2022/45**



(73) Patentinhaber: **Ivoclar Vivadent AG**
**9494 Schaan (LI)**

(72) Erfinder:
- **MOSZNER, Norbert**
  **9495 Triesen (LI)**
- **Angermann, Jörg**
  **7320 Sargans (CH)**
- **Lamparth, Iris**
  **9472 Grabs (CH)**
- **CATEL, Yohann**
  **9497 Buch (CH)**
- **RIST, Kai**
  **6800 Feldkirch (AT)**

(74) Vertreter: **Uexküll & Stolberg**
**Partnerschaft von**
**Patent- und Rechtsanwälten mbB**
**Beselerstraße 4**
**22607 Hamburg (DE)**



(56) Entgegenhaltungen:
**EP-A1- 2 492 289** **EP-A1- 3 215 353**
**EP-A1- 3 564 206** **EP-A1- 3 564 282**
**EP-B1- 3 215 353** **US-A1- 2017 333 167**
**US-B2- 9 333 148**


Bemerkungen:
Die Akte enthält technische Angaben, die nach dem Eingang der Anmeldung eingereicht wurden und die nicht in dieser Patentschrift enthalten sind.


Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).


EP 4 085 893 B1

(52) Gemeinsame Patentklassifikation (CPC): (Forts.)

C-Sets
**A61K 6/887, C08L 33/08;**
**A61K 6/887, C08L 33/10;**
**C08F 293/005, C08F 220/20, C08F 222/22**

**Beschreibung**

Die vorliegende Erfindung betrifft radikalisch polymerisierbare Zusammensetzungen, die sich besonders als Dentalwerkstoff zur Herstellung von dentalen Formkörpern, wie künstlichen Zähnen, Zahnprothesen, Inlays, Onlays, Splints (Aufbissschienen), Kronen, Brücken, Verblendmaterialien und orthodontischen Vorrichtungen durch generative Verfahren eignen.

Klassische dentale Polymerisationssysteme bestehen meistens aus einer Mischung von transparenten flüssigen Monomeren, Initiator-Komponenten, Stabilisatoren und Pigmenten (J. Viohl, K. Dermann, D. Quast, S. Venz, Die Chemie zahnärztlicher Füllungskunststoffe, Carl Hanser Verlag, München-Wien1986, 21-27). Dabei werden als Monomere für den Aufbau von Polymernetzwerken, z.B. in Füllungsmaterialien, meistens Mischungen von Dimethacrylaten eingesetzt (vgl. A. Peutzfeldt, Resin composites in dentistry: The monomer systems, Eur. J. Oral. Sci. 105 (1997) 97-116; N. Moszner, T. Hirt, New Polymer-Chemical Developments in Clinical Dental Polymer Materials: Enamel-Dentin Adhesives and Restorative Composites, J. Polym. Sci. Part A: Polym. Chem. 50 (2012) 4369-4402). Beispiele hierfür sind die hochviskosen Dimethacrylate 2,2-Bis[4-(2-hydroxy-3-methacryloyloxypropyl)phenyl]propan (Bis-GMA) und 1,6-Bis-[2-methacryloyloxyethoxycarbonylamino]-2,4,4-trimethylhexan (UDMA) sowie die als Verdünnermonomere genutzten niedrigviskosen Dimethacrylate Bismethacryloyloxymethyltricyclo[5.2.1.]decan (TCDMA), Decandiol-1,10-dimethacrylat ($D_3MA$) und Triethylenglycoldimethacrylat (TEGDMA). Demgegenüber wird für Zahnprothesen hauptsächlich das monofunktionelle Monomer Methylmethacrylat (MMA) eingesetzt, das zwar niedrigviskos aber sehr flüchtig ist.

Je nach Anwendungsgebiet werden weitere Additive und zur Auslösung der radikalischen Polymerisation geeignete Initiatoren zugesetzt. Zur Polymerisationsauslösung durch Licht werden Photoinitiatoren für den sichtbaren Bereich verwendet, die mit Blaulicht Radikale bilden und die sich durch eine gute Durchhärtungstiefe und sehr gute Bleicheigenschaften auszeichnen. Prothesenmaterialien enthalten als eine wesentliche Komponente pulverförmiges Polymethylmethacrylat (PMMA), das mit MMA eine Paste bildet. Die Aushärtung erfolgt thermisch oder mit Redoxinitiatoren. Bei Füllungskompositen und Befestigungszementen sorgen hohe Anteile an anorganischen Füllstoffen für eine hohe Biegefestigkeit und Oberflächenhärte, wobei sich Füllstoffe aber nachteilig auf die Transparenz auswirken können.

Ein wesentliches Problem radikalischer Methacrylatpolymerisate ist der Polymerisationsschrumpf ($\Delta V_P$), d.h. die bei der Polymerisation auftretende Volumenkontraktion der eingesetzten Methacrylatmonomere, was z.B. zu einer sehr nachteiligen Randspaltbildung bei Füllungskompositen führen kann und bei Prothesenmaterialen die Dimensionsstabilität nachteilig beeinflusst ($\Delta V_P$ beträgt z.B. bei reinem MMA 21,0 Vol.-%).

Ein weiterer Nachteil von PMMA- oder Dimethacrylatpolymerisaten ist die große Sprödigkeit der Materialien. Die geringe Bruchzähigkeit ist eine inhärente Eigenschaft des amorphen PMMA-Glases und wird bei den durch Dimethacrylatmischungen gebildeten Polymernetzwerken vor allem durch deren sehr unregelmäßige Netzwerkstruktur verursacht.

In den letzten Jahren haben additive Fertigungsverfahren zur Herstellung von dentalen Formkörpern zunehmendes Interesse und verbreitet Anwendung gefunden. Bei additiven Fertigungsverfahren, die auch als generative Fertigungsverfahren bezeichnet werden, werden 3D-Formkörper ausgehend von einem CAD-Datensatz schichtenweise aus polymerisierbaren Materialien erzeugt, wobei das Härten der Schichten durch gesteuerte Belichtung erfolgt. Bei der Stereolithographie (SL) wird ein UV-Laser als Lichtquelle verwendet. Beim DLP-Verfahren (Digital Light Processing) verwendet man ein projizierbares Bild zur schichtenweise Aushärtung des Photopolymersationsharzes.

Baumaterialien zur generativen Herstellung von dentalen Formkörpern müssen im Hinblick auf die Besonderheiten dieses Verfahrens unterschiedliche Anforderungen erfüllen. Sie sollen eine niedrige Viskosität und eine hohe Transparenz aufweisen und nach der Härtung gute mechanische Eigenschaften haben. Die im Stand der Technik offenbarten Materialien sind hinsichtlich bestimmter Eigenschaften optimiert, oft auf Kosten anderer Eigenschaften.

Die Verwendung mono- und polyfunktioneller Methacrylate als Harze für generative Verfahren ist Gegenstand zahlreicher Patente und Patentanmeldungen.

Die US 10,562,995 B2 offenbart Polymerisationsharze zur stereolithographischen Herstellung von Dentalprothesen, die auf Mischungen von aromatischen Di(meth)-acrylaten, die keine OH- oder COOH-Gruppen aufweisen, mit (Meth)acrylmonomeren basieren, die mindestens eine OH- oder COOH-Gruppe enthalten.

Die US 10,568,814 B2 offenbart photopolymerisierbare Zusammensetzungen zur Herstellung von künstlichen Zähnen und Prothesenbasen durch 3D-Druck, die nach der Härtung eine hohe Biegefestigkeit und ein hohes E-Modul aufweisen sollen. Die Materialien basierend auf einer Mischung von ethoxyliertem Bisphenol-A-Dimethacrylat, monofunktionellen Methacrylaten und Urethandimethacrylaten.

Die EP 3 020 361 A1 betrifft härtbare Zusammensetzungen für generative Fertigungsverfahren, die radikalisch polymerisierbare Polysiloxane und Disiloxane enthalten. Die Materialien sollen sich durch eine hohe Formstabilität und verbesserte Biokompatibilität auszeichnen.

Die EP 3 494 954 A1 offenbart photopolymerisierbare Zusammensetzungen zur Herstellung dentaler Prothesen, die eine Mischung von aromatischen Acrylaten mit einer Molmasse von 200 bis 800 g/mol und mindestens einem weiteren (Meth)acrylat enthalten, das aromatische und nicht aromatische Ringe enthalten kann. Die Materialien sollen sich durch

eine gute Charpy-Bruchzähigkeit auszeichnen.

Die EP 3 564 206 A1 offenbart (meth)acryloxysubstituierte Benzoesäureester, die sich als Reaktivverdünner für additive Fertigungsverfahren eignen sollen.

Dentale Formkörper müssen eine gute Bruchzähigkeit und gleichzeitig eine gute Biegefestigkeit und ein hohes E-Modul aufweisen. Eine Verbesserung der Bruchzähigkeit lässt sich durch innere Weichmachung erzielen, z.B. durch die Zugabe von flexiblen Monomeren. Diese haben den Nachteil, dass sie die Biegefestigkeit und das E-Modul der Polymerisate deutlich verringern. Nach dem Stand der Technik werden Polymerisationsharzen daher meist Polymerpartikel mit einer Kern-Schale-Struktur als sog. Schlagzähmodifikatoren zur Verbesserung der Bruchzähigkeit zugesetzt, die eine relative gute Biegefestigkeit und ein relativ hohes E-Modul ergeben.

Die WO 2014/078537 A1 offenbart Harzmischungen zur Herstellung dentaler Formkörper durch 3D-Druckverfahren auf der Basis von mono- und multifunktionellen Methacrylaten, die Silicon-Acrylat-basierende Schlagzähmodifikatoren mit Kern-Schale-Struktur zur Verbesserung der Schlag- und Bruchzähigkeit erhalten,.

Die US 2018/0000570 A1 betrifft Baumaterialien auf der Basis von mono- und multifunktionellen (Meth)acrylaten zur generativen Herstellung dentaler Bauteile. Die Baumaterialien enthalten Gummipartikel auf Silikon-Acryl-Basis mit Kern-Schale-Struktur (Produkt S2006, Mitsubishi Rayon Co.) als Schlagzähmodifikatoren und Oligomere, die durch Umsetzung von Trimethyl-1,6-diisocyanat, Bisphenol-A-propoxylat und 2-Hydroxyethylmethacrylat (HEMA) hergestellt werden. Die gehärteten Bauteile sollen gute mechanische und physikalische Eigenschaften sowie eine gute Bioverträglichkeit aufweisen.

Die US 10,299,896 B2 und die US 2019/0053883 A1 offenbaren durch generative Verfahren hergestellte dentale Bauteile, die mindestens zwei Schichten aus unterschiedlich zusammengesetzten Baumaterialien aufweisen. Dabei wird eine Schicht durch ein Material gebildet, das Oligomere, die durch Reaktion von Zwischenprodukten mit endständigen Isocyanatgruppen mit hydroxylbasierten Methacrylaten erhalten werden, eine polymerisierbare Acrylverbindung und einen Schlagzähmodifikator enthält. Mindestens eine weitere Schicht wird durch ein Material gebildet, das ein Urethanmonomer, ein Glycoldimethacrylat und Füllstoff enthält. Die Kombination von Materialen mit unterschiedlichen mechanischen und physikalischen Eigenschaften soll zur Anpassung der Bauteile an unterschiedliche Anforderungen vorteilhaft sein. Als Schlagzähmodifikatoren werden handelsübliche Polymere mit Kern-Schale-Struktur verwendet, wie z.B. das Produkt M570 der Firma Kaneka.

Die EP 3 564 282 A1 offenbart härtbare Zusammensetzungen für Hochtemperatur-Photopolymerisationsverfahren, die ein oligomeres Urethandimethacrylat als Glasübergangstemperaturmodifikator, ein (Poly-)Carbonat-(Poly-)-Urethan-Dimethacrylat als Zähigkeitsmodifikator und ggf. Core-Shell-Partikel enthalten. Sie sollen gute thermomechanische Eigenschaften und eine gute Bioverträglichkeit aufweisen und sich zur Herstellung kieferorthopädischer Vorrichtungen eignen. EP 2 492 289 A1 offenbart polymerisierbare zahnmedische Zusammensetzung, die ein Acrylblockcopolymer (a) mit mindestens einem Polymerblock A, der hauptsächlich eine (Meth)acrylsäureestereinheit enthält, und mindestens einem Polymerblock B, der hauptsächlich eine Acrylsäureestereinheit enthält.

Die Wirkungsweise von Schlagzähmodifikatoren mit Kern-Schale-Struktur beruht auf einer Wechselwirkung der Spitze eines sich bildenden Risses mit den Kern-Schale-Polymer-Partikeln. Diese Partikel weisen einen relativ weichen Polymerkern und eine harte Polymerschale auf. Trifft eine Rissspitze auf einen solchen Partikel kommt es zur Hohlraumbildung im Kern und zur Abtrennung der Polymerschale von der polymerisierten Harzmatrix sowie des Kerns von der Schale und damit zur Raumbildung (Kavitation) für plastische Deformationsflächen. Ein wesentlicher Nachteil für dentale Anwendungen ist, dass Kern-Schale-Polymere (CSP) die Transparenz der Materialien deutlich vermindern, was sich nachteilig auf den stereolithographischen Bauprozess auswirkt.

Der Erfindung liegt die Aufgabe zugrunde, Materialien zur Herstellung dentaler Formkörper zur Verfügung zu stellen, die ein für generative Verfahren optimiertes Eigenschaftsprofil aufweisen. Die Materialien sollen insbesondere eine hohe Transparenz in Kombination mit einer guten Bruchzähigkeit und hohen Brucharbeit haben. Sie sollen außerdem eine niedrige Viskosität und gute mechanische Eigenschaften nach Wasserlagerung sowie eine gute Bioverträglichkeit zeigen.

Erfindungsgemäß wird diese Aufgabe durch radikalisch polymerisierbare Dentalwerkstoffe gelöst wie in den Ansprüchen definiert, die mindestens ein ABA- oder AB-Blockcopolymer enthalten. Die Dentalwerkstoffe enthalten außerdem mindestens ein monofunktionelles, radikalisch polymerisierbares Monomer (a) und auch mindestens ein radikalisch polymerisierbares Urethandi(meth)acrylat-Telechel (b).

Die erfindungsgemäßen Dentalwerkstoffe weisen vorzugsweise die folgende Zusammensetzung auf:

(a) 30 bis 70 Gew.-%, vorzugsweise 30 bis 61 Gew.-% und besonders bevorzugt 40 bis 60 Gew.-% mindestens eines aromatischen, bicyclischen oder tricyclischen Mono(meth)acrylats,

(b) 20 bis 60 Gew.-%, vorzugsweise 30 bis 55 Gew.-% und besonders bevorzugt 33 bis 55 Gew.-% mindestens eines Urethandi(meth)acrylat-Telechels mit einer zahlenmittleren Molmasse von 750 bis 2000 g/mol,

(c) 0 bis 30 Gew.-%, vorzugsweise 0 bis 20 Gew.-% und besonders bevorzugt 0 Gew.-% Di(meth)acrylatmonomer(e),

(d) 1 bis 12 Gew.-%, vorzugsweise 2 bis 12 Gew.-% und besonders bevorzugt 2 bis 10 Gew.-% mindestens eines

ABA- und/oder AB-Blockcopolymers, wobei der oder die A-Blöcke homogen mischbar und der B-Block nicht homogen mischbar mit der Mischung der Komponenten (a) bis (c) sind und

(e) 0,1 bis 5,0 Gew.-%, vorzugsweise 0,2 bis 4,0 Gew.-% und besonders bevorzugt 0,3 bis 3,0 Gew.-% mindestens eines Initiators für die radikalische Polymerisation.

Wenn nicht anders angegeben beziehen sich alle Gewichtsprozentangaben hierin auf die Gesamtmasse des Werkstoffs.

Die erfindungsgemäßen Dentalwerkstoffe enthalten als **Komponente (a)** vorzugsweise mindestens ein aromatisches, bicyclisches oder tricyclisches Mono(meth)acrylat der Formel (I)

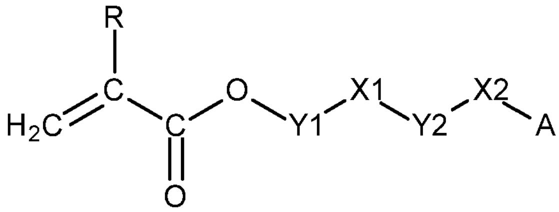

**Formel I**

in der die Variablen die folgenden Bedeutungen haben:

- A eine aromatische Gruppe mit 6 bis 15 Kohlenstoffatomen oder eine bicyclische oder tricyclische aliphatische Gruppe mit 7 bis 10 Kohlenstoffatomen ist, wobei A unsubstituiert oder durch eine oder mehrere $C_1$-$C_5$-Alkyl-Gruppen, $C_1$-$C_5$-Alkoxy-Guppen und/oder Chloratome substituiert sein kann;
- R Wasserstoff oder Methyl;
- $X^1$, $X^2$ unabhängig voneinander jeweils entfällt oder eine Ether-, Ester- oder Urethangruppe, wobei $X^1$ entfällt wenn $Y^1$ entfällt und wobei $X^2$ entfällt wenn $Y^2$ entfällt;
- $Y^1$, $Y^2$ unabhängig voneinander jeweils entfällt oder ein verzweigter oder vorzugsweise linearer aliphatischer Kohlenwasserstoffrest mit 1 bis 10 Kohlenstoffatomen, der durch 1 bis 3 Sauerstoffatome unterbrochen sein kann.

(Meth)acrylat steht hierin für Acrylat, Methacrylat oder eine Mischung davon, wobei in allen Fällen die Bedeutung Methacrylat bevorzugt ist.

Alle hierin gezeigten Formeln erstrecken sich nur auf solche Verbindungen, die mit der chemischen Valenzlehre vereinbar sind. Der Hinweis, dass ein Rest z.B. durch ein oder mehrere Sauerstofftome unterbrochen ist, ist so zu verstehen, dass diese Atome jeweils in die Kohlenstoffkette des Restes eingeschoben werden. Diese Atome sind damit beidseitig durch C-Atome begrenzt und können nicht endständig sein. $C_1$-Reste können nicht verzweigt oder unterbrochen sein. Unter aromatischen Kohlenwasserstoffresten werden der üblichen Nomenklatur entsprechend auch solche Reste verstanden, die aromatische und nicht aromatische Gruppen enthalten. Ein bevorzugter aromatischer Rest ist beispielsweise 2,2-Diphenylpropan.

Die für die einzelnen Variablen angegebenen bevorzugten, besonders bevorzugten und ganz besonders bevorzugten Definitionen können jeweils unabhängig voneinander ausgewählt werden. Verbindungen, in denen alle Variablen die bevorzugten, besonders bevorzugten und ganz besonders bevorzugten Definitionen aufweisen, sind naturgemäß erfindungsgemäß besonders geeignet.

Bevorzugte aromatische Gruppen A sind Benzol, Biphenyl und 2,2-Diphenylpropan:

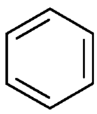 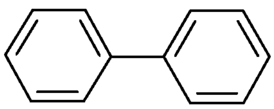 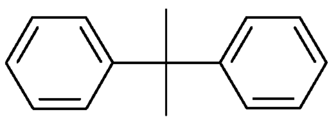

Bevorzugte bicyclische aliphatische Gruppen A sind Bicyclo[4.4.0]decan, Bicyclo[4.3.0]nonan, Bicyclo[2.2.2]octan und Bicyclo[2.2.1]heptan:

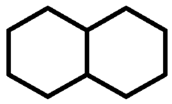 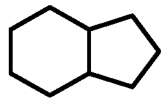 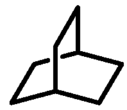 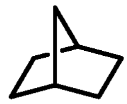

Eine bevorzugte tricyclische aliphatische Gruppe A ist Tricyclo[5.2.1.0$^{2,6}$]decan:

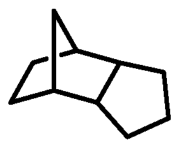

Bevorzugte aromatische Mono(meth)acrylate (a) sind 2-Phenoxyethyl(meth)acrylat, 2-(o-Biphenyloxy)ethyl(meth)acrylat, 2-Hydroxy-3-phenoxypropyl-(meth)acrylat, 2-[(Benzyloxycarbonyl)-amino]-ethyl(meth)acrylat, 2-[(Benzylcarbamoyl)-oxy]-ethyl-(meth)acrylat, 1-Phenoxypropan-2-yl-(meth)acrylat und 2-(p-Cumylphenoxy)-ethyl-(meth)acrylat. Besonders geeignete aromatische Mono(meth)acrylate sind 2-Phenoxyethyl(meth)acrylat, 2-(o-Biphenyloxy)ethyl(meth)acrylat, 2-Hydroxy-3-phen-oxypropyl(meth)acrylat, 2-[(Benzyloxycarbonyl)amino]-ethyl(meth)acrylat, 1-Phenoxy-propan-2yl-(meth)acrylat, 2-(Benzyloxy)ethyl(meth)acrylat, 3-Phenoxybenzyl(meth)-acrylat, Phenoxypropyl(meth)acrylat, 2-Benzyloxyethyl(meth)acrylat, 2-Benzoyloxy-ethyl(meth)acrylat, 2-(Meth)acryloyloxybenzoesäuremethylester, 2-Phenylethyl-(meth)acrylat und/oder 2-(p-Cumylphenoxy)ethyl(meth)acrylat.

Bevorzugte bi- oder tricylische Mono(meth)acrylate (a) sind Tricylodecan-(meth)acrylat, Tricyclodecanmethyl(meth)acrylat und insbesondere 4,7,7-Trimethylbicyclo[2.2.1]heptanyl(meth)acrylat.

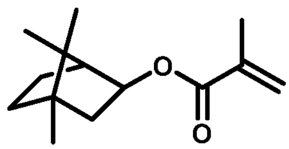 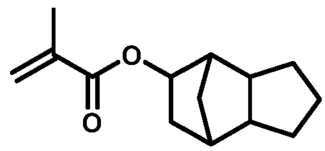

Die erfindungsgemäß verwendeten aromatischen, bicyclischen oder tricyclischen Monomethacrylate der Formel (I) zeichnen sich durch eine gute radikalische Polymerisationsfähigkeit aus. Darüber hinaus haben die Polymerisate dieser Monomethacrylate einen vergleichsweise geringen Polymerisationsschrumpf und gute mechanische Eigenschaften. Aufgrund ihrer relativ hohen Molmasse (150 bis 350 g/mol) und ihrer relativ unpolaren Struktur haben die Mono(meth)acrylate der Formel (I) außerdem eine geringe Flüchtigkeit und eine vergleichsweise geringe Viskosität.

Die erfindungsgemäßen Dentalwerkstoffe enthalten als **Komponente (b)** mindestens ein Urethandimethacrylat-Telechel mit einer Molmasse von 750 bis 2000 g/mol. Die Komponente (b) enthält zwei radikalisch polymerisierbare Gruppen und wirkt bei der Polymerisation der erfindungsgemäßen Werkstoffe damit als Vernetzer, d.h. sie führt zur Bildung von Polymernetzwerken. Aufgrund der relativ hohen Molmasse der Komponente (b) werden Polymerisate mit einer geringeren Netzwerkdichte und geringem Polymerisationsschrumpf erhalten.

Wenn nicht anders angegeben, handelt es sich hierin bei der Molmasse von Oligomeren und Polymeren um die zahlenmittlere Molmasse, deren Absolutwerte man mit den bekannten Methoden der Gefrierpunktserniedrigung (Kryoskopie), der Siedepunktserhöhung (Ebullioskopie) oder aus der Erniedrigung des Dampfdrucks (Dampfdruckosmometrie) bestimmen kann. Vorzugsweise wird die zahlenmittlere Molmasse von Oligomeren und Polymeren mittels Gel-Permeations-Chromatographie (GPC) bestimmt. Dabei handelt es sich um eine Relativmethode bei der die Moleküle aufgrund ihrer Größe, genauer gesagt aufgrund ihres hydrodynamischen Volumens, getrennt werden. Die Bestimmung der absoluten Molmasse erfolgt durch Kalibrierung mit bekannten Standards.

Urethandimethacrylat-Telechele (b) werden vorzugsweise durch Umsetzung von Diisocyanaten mit Diolen (HO-DA-OH) und anschließender Reaktion der $\alpha,\omega$-isocyanat-funktionalisierten Urethantelechele mit HEMA oder HPMA erhalten. DA steht vorzugsweise für einen aromatischen oder aliphatischen Kohlenwasserstoffrest mit 6 bis 33 Kohlenstoffatomen, vorzugsweise einen divalenten polycyclischen Kohlenwasserstoffrest, insbesondere einen o-Diphenyl-, p-Diphenyl- oder Bisphenol A-Rest, oder eine verzweigte oder vorzugsweise lineare $C_2$-$C_{18}$-Alkylengruppe. Die Kohlenwasserstoffreste können ein oder mehrere O-Atome und/oder S-Atome enthalten, wobei O-Atome bevorzugt sind.

Erfindungsgemäße Diole der Formel HO-DA-OH sind ethoxyliertes- oder propoxyliertes Bisphenol-A, o-Diphenyl oder p-Diphenyl mit 2 bis 6 Ethoxy- oder Propoxy-Gruppen sowie $C_2$-$C_{18}$-Alkandiole, die 1 bis 4 O- oder S-Atome in der Kohlenstoffkette enthalten können, cyclische oder polycyclische aliphatische Diole. Bevorzugte Diole sind ethoxyliertes- oder propoxyliertes Bisphenol-A mit 2, 3 oder 4 Ethoxy- oder Propoxygruppen, Hexan-1,6-diol, Octan-1,8-diol, Nonan-1,9-diol, Decan-1,10-diol oder Dodecan-1,12-diol, Tetra- oder Pentaethylenglycol. Ganz besonders bevorzugt sind ethoxyliertes- oder propoxyliertes Bisphenol-A mit 2 oder 3 Ethoxy- oder Propoxygruppen, Decan-, Undecan- oder

Dodecandiol, Cyclohexandiol, Norbornandiol, Tricyclodecandiol und Tricyclodecandimethanol (Octahydro-4,7-methano-1H-indendimethanol).

Bevorzugte Diisocyanate sind Hexamethylen-1,6-diisocyanat (HMDI), 2,2,4-Tri-methylhexamethylen-1,6-diisocyanat (TMDI), 1-Isocyanato-3-isocyanatomethyl-3,5,5-trimethylcyclohexan (Isophorondiisocyanat, IPDI), m-Tetramethylxylylendiisocyanat (1,3-Bis(2-isocyanato-2-ppropyl)benzol, TMXDI), Toluol-2,4-diisocyanat (TDI), Diphenylmethan-4,4'-diisocyanat (MDI) und 1-Isocyanato-4-[(4-isocyanatocyclohexyl)methyl]cyclohexan ($H_{12}$MDI), wobei IPDI besonders bevorzugt ist.

Erfindungsgemäß erforderlich sind Telechele gemäß der allgemeinen Formel (II)

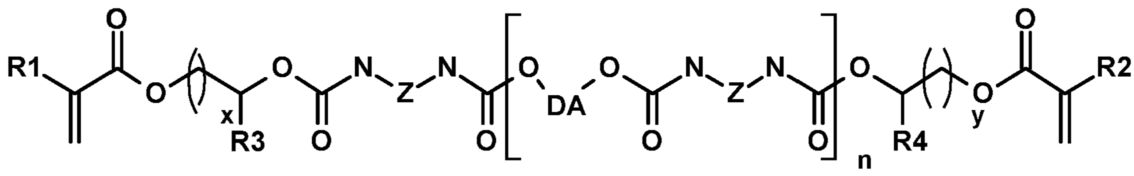

**Formel II**

in der die Variablen die folgenden Bedeutungen haben:

$R^1$, $R^2$ unabhängig voneinander jeweils H oder Methyl, vorzugsweise Methyl,

$R^3$, $R^4$ unabhängig voneinander jeweils H oder Methyl, vorzugsweise Methyl,

x, y unabhängig voneinander jeweils eine ganze Zahl von 1 bis 11, vorzugsweise 1 bis 5,

n 1, 2 oder 3, vorzugsweise 1

Z

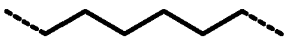 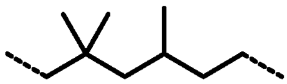 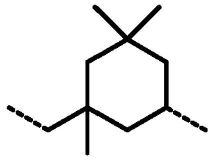 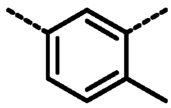

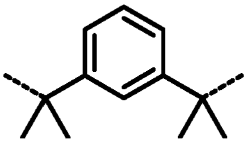 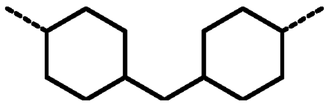 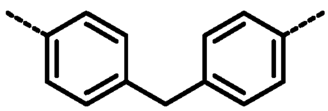

vorzugsweise :

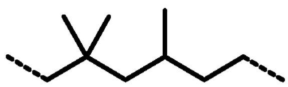 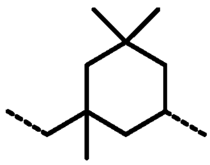 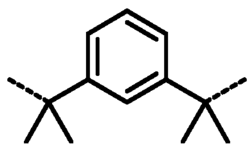

DA ein Strukturelement, das sich aus den Diolen HO-DA-OH durch Abspaltung der Wasserstoffatome der beiden Hydroxylgruppen ableitet.

Die erfindungsgemäße Urethandimethacrylat-Telechele zeichnen sich durch eine gute radikalische Polymerisationsfähigkeit aus. Außerdem verleihen sie den gehärteten Materialien gute kohäsive Eigenschaften.

Monofunktionelle Methacrylate (a), Urethandimethacrylat-Telechele (b) und ggf. weitere radikalisch polymerisierbare Monomere werden vorzugsweise in einem solchen Mengenverhältnis eingesetzt, dass vernetzte Polymere mit einer Netzwerkdichte von unter $v_c$ = 300 bis 5000 mol/$m^3$, besonders bevorzugt 400 bis 3000 mol/$m^3$ erhalten werden. Die Vernetzungsdichte wird maßgeblich durch das Verhältnis von vernetzenden zu monofunktionellen Monomeren bestimmt. Erfindungsgemäß bevorzugt sind Dentalwerkstoffe, in denen der Molenbruch der vernetzenden Monomeren in einem Bereich von 0,1 bis 0,6 und besonders bevorzugt 0,15 bis 0,45 liegt. Zur Berechnung des Molenbruchs werden alle radikalisch polymerisierbaren Komponenten der erfindungsgemäßen Werkstoffe herangezogen, d.h. insbesondere die Komponenten (a) bis (c) und ggf. weitere radikalisch polymerisierbare Monomere. Unter vernetzenden Monomeren werden alle radikalisch polymerisierbaren Komponenten verstanden, die zwei oder mehr radikalisch polymerisierbare Gruppen aufweisen, d.h. insbesondere die Komponenten (b) und (c). Vernetzende Monomere werden auch als polyfunktionelle Monomere bezeichnet. Monofunktionelle Monomere sind Monomere mit nur einer radikalisch polymerisierbaren Gruppe.

Die Netzwerkdichte entspricht der Zahl der Netzknoten (in mol) je Volumeneinheit und lässt sich durch

dynamisch-mechanische Messungen aus dem Plateauwert des Speichermoduls G' im elastischen Bereich berechnen. Die Glasübergangstemperatur $T_g$ und die Netzwerkdichte $v_c$ werden mit einem Rheometer bestimmt, vorzugsweise einem Anton Paar Rheometer MCR301. Hierzu werden Speicher- und Verlustmodul eines Prüfkörpers (25 x 5 x 1 mm, längs eingespannt) zwischen 25°C und 250°C vermessen (Frequenz 1 Hz, Deformation 0,05 %, Heizrate 2K / min). $T_g$ ergibt sich dabei als das Maximum des Verlustfaktors tan $\delta$ (Verhältnis von Verlustmodul zu Speichermodul). Die Netzwerkdichte wird nach der Formel $v_c = G'/(RT)$ berechnet, mit G' als dem Speichermodul bei der Temperatur $T_g$ + 50 K, R als der allgemeinen Gaskonstante und T als der Temperatur bei $T_g$ + 50 K in Kelvin.

Die erfindungsgemäßen Dentalwerkstoffe können zur weiteren Einstellung der Vernetzungsdichte und zur Beeinflussung der mechanischen Eigenschaften der Polymerisate neben den Komponenten (a) und (b) zusätzlich weitere **Di(meth)acrylatmonomere (c)** enthalten.

Bevorzugte Di(meth)acrylate (c) sind Bisphenol-A-dimethacrylat (Bis-GMA, ein Additionsprodukt aus Methacrylsäure und Bisphenol-A-diglycidylether), ethoxyliertes- oder propoxyliertes Bisphenol-A-dimethacrylat, wie z.B. das Bisphenol-A-dimethacrylat SR-348c (Sartomer) mit 3 Ethoxygruppen, 2,2-Bis[4-(2-methacryloxypropoxy)-phenyl]propan (UDMA, ein Additionsprodukt aus HEMA und TMDI), V380 (ein Additionsprodukt aus einer Mischung von 0,7 mol 2-Hydroxyethylmethacrylat und 0,3 mol 2-Hydroxypropylmethacrylat mit 1 mol $\alpha,\alpha,\alpha',\alpha'$-Tetramethyl-m-xylylen-diisocyanat), Bis(methacryloyloxymethyl)tricyclo-[5.2.1.0$^{2,6}$]decan (DCP), Di-, Tri- oder Tetraethylenglycoldimethacrylat, Trimethylolpropantrimethacrylat, Pentaerythrittetra(meth)-acrylat, sowie Glycerindi- und Glycerintrimethacrylat, 1,4-Butandioldimethacrylat, 1,10-Decandioldimethacrylat ($D_3MA$) und 1,12-Dodecandioldimethacrylat.

Die Di(meth)acrylatmonomere (c) zeichnen sich durch ein relativ geringes MolGewicht aus. Erfindungsgemäß bevorzugt sind Di(meth)acrylate (c) mit einem MolGewicht im Bereich von 200 bis 800 g/mol, vorzugsweise 220 bis 650 g/mol. Wegen des im Vergleich zu den Urethandi(meth)acrylat-Telechelen (b) geringen MolGewichts bewirken die Di(meth)acrlatmonomere (c) eine relativ starke Vernetzung der Polymerisate und führen so zu einer hohen Netzwerkdichte, die sich nachteilig auf die Bruchzähigkeit auswirken kann. Der Anteil weiterer Di(meth)acrylate ist daher auf maximal 30 Gew.-%, vorzugsweise maximal 10 Gew.-% beschränkt. Gemäß einer besonders bevorzugten Ausführungsform enthalten die erfindungsgemäßen Dentalwerkstoffe als Vernetzer ausschließlich die Urethandi(meth)acrylat-Telechele (b).

Außerdem können die erfindungsgemäßen Dentalwerkstoffe neben der Komponente (a) weitere Mono(meth) acrylate enthalten. Der Anteil weitere Mono(meth)acrylate liegt vorzugsweise unter 10 Gew.-%, wobei Werkstoffe, die keine weiteren Mono(meth)acrylate enthalten, besonders bevorzugt sind.

Die erfindungsgemäßen Dentalwerkstoffe enthalten als **Komponente (d)** mindestens ein ABA- und/oder ein AB-Blockcopolymer. Unter Blockcopolymeren werden Makromoleküle verstanden, die aus zwei oder mehr kovalent miteinander verbundenen Homopolymerblöcken bestehen.

Erfindungsgemäß bevorzugte Blockcopolymere lassen sich mit den bekannten Methoden der lebenden bzw. kontrollierten Polymerisation herstellen, beispielsweise durch radikalische oder ionische (anionische und kationische) Polymerisation, wobei die kontrollierte radikalische Polymerisation und die lebende anionische Polymerisation bevorzugt sind. Blockcopolymere können aber auch durch die Verknüpfung von Endgruppen von Homopolymeren erhalten werden. Die erfindungsgemäß verwendeten Blockcopolymere können als Di- und Triblockcopolymere vorliegen.

AB-Blockcopolymere lassen sich beispielsweise herstellen, indem ein A-Block mit endständiger OH-Gruppe durch Veresterung mit einem B-Block verknüpft wird, der eine COOH-Gruppe aufweist. Endgruppenfunktionalisierte Homopolymerblöcke lassen sich relativ einfach mit den Methoden der geregelten radikalischen Polymerisation oder durch Endcapping bei der anionischen Polymerisation herstellen.

Beispielsweise wird das Monomer A anionisch polymerisiert und durch Endcapping eine OH-Gruppe eingeführt. Die OH-Endgruppe lässt sich dann z.B. mit $\alpha$-Bromisobuttersäure verestern. Die dabei erhaltene Brom-Endgruppe fungiert dann als Startzentrum für die Bildung des B-Blocks durch ATRP (Atom Transfer Radical Polymerization) von Monomer B, initiiert durch Metallkomplexe beispielsweise von Cu(I), Ru(I) oder Fe(II).

Triblockcopolymere lassen sich auf analoge Weise herstellen. Beispielsweise wird durch anionische Polymerisation von Monomer B über einen Dianionen-Mechanismus ein B-Block hergestellt. Der gebildete B-Mittelblock trägt beidseitig jeweils eine Anion-Endgruppe, die die anionische Polymerisation von Monomer A unter Bildung der beiden A-Blöcke initiiert (Methode 1). Die Veresterung eines telechelen B-Blocks, der an beiden Enden jeweils eine geeignete funktionelle Gruppe, z.B. eine OH-Gruppe trägt, mit zwei A-Blöcken, die nur einseitig, z.B. mit einer COOH-Gruppe, funktionalisiert sind, ergibt ABA-Triblockcopolymere (Methode 2). Schließlich lassen sich OH-telechele Homopolymere von Monomer B mit $\alpha$-Bromisobuttersäure verestern. Die beiden so gebildeten Brom-Endgruppen im Homopolymerblock B lassen sich dann als Startzentrum für die Bildung der beiden A-Blöcke durch ATRP nutzen (Methode 3).

Bei der Synthese der Blockcopolymeren lassen sich auch end- oder seitenständig polymerisationsfähige Methacrylatgruppen einführen. Diese bewirken eine bessere Einbindung der Blockcopolymere in die gebildeten Polymernetzwerke durch radikalische Copolymerisation der Methacrylatgruppen.

Die Monomere werden vorzugsweise so gewählt, dass die A-Blöcke mit der Harzmatrix, d.h. der Mischung der Bestandteile (a) bis (c), mischbar und der B-Block nicht mit der Harzmatrix mischbar ist.

Die Mischbarkeit wird hier im Sinne der Thermodynamik in Bezug auf die Einphasigkeit verstanden. Danach wird unter einem mischbaren Polymerblock ein Polymerblock aus einem Monomer verstanden, dessen Homopolymer in der HarzMatrix löslich ist, so dass die Mischung eine Transparenz von mindestens 95 % aufweist. Ist die Mischung dagegen trüb oder opak, d.h. ist die Transparenz kleiner als 95%, so ist das Homopolymer und damit der entsprechende Polymerblock nicht mit der Harz-Matrix mischbar. Die Transparenz wird mit einem Spektrophotometer an 1 mm dicken, auf Hochglanz polierten Prüfkörpern in Transmission (D65) gemäß der Norm ISO 10526:1999 gemessen, z.B. mit einem Konika-Minolta Spektrophotometer des Typs CM-5.

Die Blockcoplymere bewirken eine deutliche Verbesserung der Bruchzähigkeit der erfindungsgemäßen Werkstoffe nach der Härtung. Es wird angenommen, dass die Unmischbarkeit der B-Blöcke der Blockcopolymere mit den übrigen Bestandteilen der erfindungsgemäßen Zusammensetzungen eine Mikrophasenseparation und damit die Ausbildung von Morphologien auf nanoskaliger Ebene bewirkt. Dabei bilden die Makromoleküle der ABA- bzw. AB-Blockcopolymeren im Monomerharz oder während der Aushärtung durch Selbstaggregation (self-assembly) sphärische oder wurmartige Phasen aus, die mit Rissspitzen interagieren können, d.h., dass Rissspitzen auf die Phasen treffen und sich die Bruchenergie dabei so in den Phasen verteilt, dass die Risse nicht weiter durch den Werkstoff wandern und sich nicht vergrößern. Die Fortpflanzung eines Risses lässt sich bei transparenten Materialen unter einem Elektronenmikroskop beobachten. In der Bruchmechanik wird der vorderste Teil des Risses als Rissspitze bezeichnet.

Erfindungsgemäß bevorzugte Blockcopolymere sind AB-Diblock- und ABA-Triblockcopolymere.

Der A-Block ist ein Polymerisat, vorzugsweise ein Oligomer, das aus einem oder mehreren der folgenden Monomere aufgebaut ist: cyclische, aliphatische Ester oder Ether, Arylenoxid, Alkylenoxid, radikalisch polymerisierbare Monomere, beispielsweise $\alpha,\beta$-ungesättigte Säuren und $\alpha,\beta$-ungesättigte Säureester. Vorzugsweise ist der Block A ein Poly(meth)acrylat-, Polylacton-, Phenylenoxid- oder Polyalkylenoxid-Oligomer. Ganz besonders bevorzugt ist der Block A ein Polymerisat von Caprolacton, 2,6-Dialkyl-1,4-phenylenoxid und insbesondere von 2,6-Dimethyl-1,4-phenylenoxid, Ethylenoxid, Propylenoxid oder (Meth)acrylaten. Der A-Block ist damit vorzugsweise ein Polycaprolacton- (PCL), Poly(2,6-dimethyl-1,4-phenylenoxid)-, Poly(ethylenoxid)-, Poly(propylenoxid)- oder Poly(meth)acrylat-Oligomer.

Der B-Block ist vorzugsweise ein Polysiloxan- und/oder ein Polyvinyl- und/oder ein Polyalken- und/oder ein Polydien-Oligomer. Besonders bevorzugt ist der B-Block ein Polydien-Oligomer, Polyvinylalkanoat-Oligomer oder ein Polysiloxan-Oligomer gemäß der Formel $-O-(SiR^5{}_2-O)_p-$, in der

$R^5$ eine lineare $C_1$-$C_{20}$-Alkyl-, verzweigte $C_3$-$C_{12}$-Alkyl- oder $C_6$-$C_{20}$-Arylgruppe ist, wobei die einzelnen $R^5$-Reste gleich oder verschieden sein können, und

p eine Zahl von 3 bis 100, bevorzugt eine Zahl von 10 bis 50 ist.

Ganz besonders bevorzugt ist der B-Block ein Polymerisat von Dimethylchlorsilan, Cyclotri- oder Cyclotetradimethoxysilan, Isopren, Vinylacetat, Isobuten, cis-Butadien oder Ethylen. Der B-Block ist damit vorzugsweise ein Poly(dimethylsiloxan)- (PDMS), Poly(isopren)-, Poly(vinylacetat)-, Poly(isobuten)-, cis-Poly(butadien)- oder Poly-(ethylen)-Oligomer.

Die B-Blöcke zeichnen sich durch eine relativ hohe Flexibilität aus. Unter flexiblen Blöcken werden Blöcke verstanden, die aus Monomeren gebildet werden, deren Homopolymeren eine Glasübergangstemperatur $T_G$ unter 50 °C, vorzugsweise unter 0 °C und ganz besonders bevorzugt im Bereich von -30 bis -110 °C haben. Blockcopolymere mit flexiblen Blöcken verbessern die Bruchzähigkeit, beinträchtigen die Biegefestigkeit und das E-Modul der Polymerisate aber deutlich weniger als innere Weichmacher.

Erfindungsgemäß bevorzugt sind Polyester-Polysiloxan-Blockcopolymere gemäß der folgenden allgemeinen Formel:

$$(PCL)_q\text{-b-}(PDMS)_r\text{-b-}(PCL)_q$$

ist der

q jeweils eine Zahl von 5 bis 40, bevorzugt 10 bis 20, ist und

r eine Zahl von 10 bis 100, bevorzugt 30 bis 60, ist.

$(PCL)_q$ steht für Polycaprolacton, das aus q Caprolactonmonomeren aufgebaut ist, und $(PDMS)_r$ für Poly(dimethylsiloxan), das aus r Dimethylsiloxanmonomeren aufgebaut ist. Der Buchstabe b steht für *block.*

Weiter bevorzugt sind Poly(meth)acrylat-Polysiloxan-Blockcopolymere, die als A-Block einen Polymethylmethacrylat-Rest und als B-Block einen Polysiloxan-Rest enthalten, wobei der Polysiloxan-Rest vorzugsweise wie oben definiert und ganz besonders bevorzugt ein Poly(dimethylsiloxan)-Rest ist.

Besonders bevorzugt sind die ABA-Triblockcopolymeren PCL-b-PDMS-b-PCL und PMMA-b-PDMS-b-PMMA mit einem Molverhältnis A:B von 0,1 bis 5 und mit einer Molmasse von bevorzugt 3 bis 25 kDa, besonders bevorzugt 4 bis

20 kDa und ganz besonders bevorzugt 5 bis 10 kDa. Ein bevorzugtes Blockcopolymer ist PCL-b-PDMS-b-PCL, wobei die PDMS-Blöcke eine Molmasse von ca. 3200 g/mol und die PCL-Blöcke eine Molmasse von jeweils ca. 1600 g/mol aufweisen. PCL steht für Polycaprolacton, PDMS für Poly(dimethylsiloxan) und PMMA für Polymethylmethacrylat.

Das oder die Blockcopolymeren werden vorzugsweise in einer Menge von 1 bis 12 Gew.-%, besonders bevorzugt in einer Menge von 2 bis 10 Gew.-% und ganz besonders bevorzugt 3 bis 8 Gew.-% eingesetzt, bezogen auf das Gesamtgewicht des Dentalwerkstoffs.

Es wurde gefunden, dass die erfindungsgemäß verwendeten Blockcopolymere die Bruchzähigkeit der Polymernetzwerke deutlich verbessern, ohne die Transparenz zu beeinträchtigen. Außerdem bewirken sie nur eine relativ geringe Viskositätserhöhung. Ein weiterer Vorteil der erfindungsgemäß verwendeten Blockcopolymere ist, dass sie sich leicht mit den übrigen Komponenten der Werkstoffe homogen mischen lassen, während das homogene Dispergieren von Kern-Schale-Polymerpartikeln deutlich aufwendiger ist. Zudem neigen Partikel zur Sedimentation, so dass Zusammensetzungen auf der Basis von Kern-Schale-Partikeln weniger stabil sind. Die Blockcopolymere lassen sich demgegenüber gut in Harzmischungen einarbeiten, so dass eine Anpassung der Werkstoffe an die geplante Anwendung und eine Einstellung der gewünschten Bruchzähigkeit und Brucharbeit problemlos möglich ist.

Die erfindungsgemäßen Dentalwerkstoffe enthalten als **Komponente (e)** mindestens einen Initiator für die radikalische Polymerisation, vorzugsweise einen Photoinitiator. Bevorzugte Photoinitiatoren sind Benzophenon, Benzoin und deren Derivate sowie α-Diketone und deren Derivate, wie 9,10-Phenanthrenchinon, 1-Phenyl-propan-1,2-dion, Diacetyl oder 4,4'-Dichlorbenzil. Besonders bevorzugt werden Campherchinon (CQ) und 2,2-Dimethoxy-2-phenylacetophenon und ganz besonders bevorzugt α-Diketone in Kombination mit Aminen als Reduktionsmittel, wie z.B. 4-(Dimethylamino)-benzoesäureester (EDMAB), N,N-Dimethylaminoethylmethacrylat, N,N-Dimethyl-sym.-xylidin oder Triethanolamin, verwendet. Bevorzugte monomolekulare Photoinitiatoren für den sichtbaren Bereich sind Monoacyltrialkyl-, Diacyldialkyl- und Tetraacylgermanium sowie Tetraacylstannane, wie z.B. Benzoyltrimethylgermanium, Dibenzoyldiethylgermanium, Bis(4-methoxybenzoyl)diethylgermanium, Tetrakis(2-methylbenzoyl)german oder Tetrakis(mesitoyl)stannan. Dabei lassen sich auch Mischungen der verschiedenen Photoinitiatoren einsetzen, wie z.B. Bis(4-methoxybenzoyl)diethylgermanium in Kombination mit Campherchinon und 4-Dimethylam inobenzoesäureethylester.

Bevorzugte Initiatoren zur Härtung der erfindungsgemäßen Dentalwerkstoffe mit UV-Licht sind Norrish-Typ-I-Photoinitiatoren, vor allem Acetophenone, z.B. 2,2-Diethoxy-1-phenylethanon, Benzoinether z.B. Irgacure 651 (Dimethylbenzilketal), Hydroxyalkylphenylacetophenone, z.B. Irgacure 184 (1-Hydroxy-cyclohexyl-phenyl-keton), Acyl- oder Bisacylphosphinoxide, z.B. Irgacure TPO (2,4,6-Trimethylbenzoyldiphenylphosphinoxid) und Irgacure 819 (Bis(2,4,6-trimethylbenzoyl)phenylphosphinoxid). Weitere bevorzugte Photoinitiatoren sind 2-Benzyl-2-(dimethylamino)-4'-morpholino-butyrophenon (Irgacure 369) und 1-Butanon-2-(dimethylamino)-2-(4-methylphenyl)methyl-1-4-(4-morpholinyl) phenyl (Irgacure 379). Besonders bevorzugte Photoinitiatoren sind Bis(4-methoxybenzoyl)diethylgermanium, Irgacure TPO und Irgacure 819 sowie Campherchinon/4-(Dimethylamino)benzoesäureester. Dabei ist es für eine Nachvergütung von Vorteil, zwei Photoinitiatoren einzusetzen, die sich durch ihre Absorptionsbereiche unterscheiden, wie z.B. Irgacure TPO und Campherchinon/4-(Dimethylamino)benzoesäureester

Die erfindungsgemäßen Dentalwerkstoffe können alternativ oder zusätzlich auch thermische Initiatoren enthalten, z.B. Azoverbindungen, wie 2,2'-Azobis(isobutyro-nitril) (AIBN) oder Azobis-(4-cyanovaleriansäure), oder Peroxide, wie Dibenzoylperoxid, Dilauroylperoxid, tert-Butylperoctoat, tert-Butylperbenzoat oder Di-(tert-butyl)-peroxid. Zur Beschleunigung der Initiierung mittels Peroxiden lassen sich auch Kombinationen mit aromatischen Aminen einsetzen. Bevorzugte Redoxsysteme sind: Kombinationen von Dibenzoylperoxid mit Aminen, wie N,N-Dimethyl-p-toluidin, N,N-Dihydroxyethyl-p-toluidin, p-Dimethylaminobenzoesäureethylester oder strukturverwandte Systeme.

Der oder die Initiatoren werden vorzugsweise in einer Gesamtmenge von 0,1 bis 5,0 Gew.-%, besonders bevorzugt 0,2 bis 4 Gew.-% und ganz besonders bevorzugt 0,3 bis 3,0 Gew.-% eingesetzt, wobei diese Mengenangaben sämtliche Initiatorbestandteile einschließen, wie z.B. Reduktionsmittel.

Die erfindungsgemäßen Dentalwerkstoffe können zur weiteren Verbesserung der Bruchzähigkeit und Schlagzähigkeit auch einen gewissen Anteil eines oder mehrerer Kern-Schale-Polymere enthalten (**Komponente (f)**). Bevorzugt sind Kern-Schale-Polymere (Core-Shell-Polymere; CSP) mit einem weichen Polymerkern, z.B. aus einem vernetzten Butylacrylat, und einer eher harten Polymerschale, z.B. PMMA. Unter weichen oder flexiblen Polymeren werden Polymere mit einer Glasübergangstemperatur $T_G$ unter 50 °C, vorzugsweise unter 0°C und ganz besonders bevorzugt im Bereich von -30 bis -110 °C verstanden. Ein bevorzugtes konkretes Beispiel ist PDMS mit einer $T_G$ von ca. -110 °C. Unter harten Polymeren werden Polymere mit einer Glasübergangstemperatur über 50 °C und bevorzugt über 80 °C verstanden. Ein bevorzugtes konkretes Beispiel ist PMMA mit einer $T_G$ von 100 °C.

Die bruchzähigkeitsmodifizierende Wirkung der CSP-Partikeln in radikalischen Dimethacrylat-Polymernetzwerken hängt vor allem von der Art der CSP-Partikel, der Partikelgröße, der Vernetzungsdichte und dem Gewichtsverhältnis von Kern zu Schale ab, das vorzugweise in einem Bereich von 1:1 bis 200:1 liegt. Die Vernetzungsdichte wird maßgeblich durch den Anteil vernetzender Monomere im Partikelkern bestimmt. Dieser liegt vorzugsweise in einem Bereich von 1 bis 10 Gew.-%, bezogen auf die Masse des Kerns. Erfindungsgemäß sind Partikel mit einer Teilchengröße von 0,20 bis 5,0 μm bevorzugt.

Erfindungsgemäß bevorzugt sind CSP-Partikel mit einem Kern aus weichen Kunststoffen wie Polybutadien, Polyisopren, Polybutylacrylat, MMA-Butadien-Styrol-Copolymeren (MBS) oder Polydimethylsiloxan, und einer Schale aus harten Kunststoffen wie PMMA oder MMA-Styrol-Copolymer. Erfindungsgemäß geeignete CSP-Partikel sind kommerziell erhältlich, z.B. von Arkema (Clearstrength), Soken (Chemisnow) oder Kaneka (z.B. M521 oder M210).

Kern-Schale-Polymere können in einer Menge von bis zu 15 Gew.-% zugesetzt werden. Nachteilig an der Verwendung von Kern-Schale-Polymeren ist, dass sie die Transparenz der Zusammensetzungen stark beeinträchtigen können, was sich bei der Photopolymerisation negativ auf die Durchhärtungstiefe und bei dentalen Formkörpern zusätzlich negativ auf die Ästhetik auswirkt. Erfindungsgemäß sind daher Werkstoffe bevorzugt, die maximal 5 Gew.-% und besonders bevorzugt keine Kern-Schale-Partikel enthalten. Bei der Einarbeitung der CSP-Partikel in den Dentalwerkstoff ist auf eine gute Dispergierung zu achten.

Zur Beeinflussung der mechanischen Eigenschaften können die erfindungsgemäßen Dentalwerkstoffe mit anorganischen partikulären **Füllstoffen (g)** verstärkt werden.

Bevorzugte anorganische Füllstoffe sind Oxide, wie $SiO_2$, $ZrO_2$ und $TiO_2$ oder Mischoxide aus $SiO_2$, $ZrO_2$, ZnO und/oder $TiO_2$, nanopartikuläre oder mikrofeine Füllstoffe, wie pyrogene Kieselsäure oder Fällungskieselsäure, Glaspulver, wie Quarz-, Glaskeramik-, Borosilikat- oder röntgenopake Glaspulver, vorzugsweise Barium- oder Strontiumaluminiumsilikatgläser, und röntgenopake Füllstoffe, wie Ytterbiumtrifluorid, Tantal(V)-oxid, Bariumsulfat oder Mischoxide von $SiO_2$ mit Ytterbium(III)-oxid oder Tantal(V)-oxid. Weiterhin können die erfindungsgemäßen Dentalwerkstoffe faserförmige Füllstoffe, Nanofasern, Whiskers oder Mischungen davon enthalten. Gemäß einer bevorzugten Ausführungsform enthalten die erfindungsgemäßen Werkstoffe keine Fluoraluminosilikatgläser, Calciumaluminiumsilikatgläser oder andere Füllstoffe, die mit organischen Säuren im Sinne einer Säure-Base-Reaktion reagieren.

Bevorzugt weisen die Oxide eine Partikelgröße von 0,010 bis 15 µm, die nanopartikulären oder mikrofeinen Füllstoffe eine Partikelgröße von 10 bis 300 nm, die Glaspulver eine Partikelgröße von 0,01 bis 15 µm, vorzugweise von 0,2 bis 1,5 µm, und die röntgenopaken Füllstoffe eine Partikelgröße von 0,2 bis 5 µm auf.

Besonders bevorzugte Füllstoffe sind Mischoxide aus $SiO_2$ und $ZrO_2$, mit einer Partikelgröße von 10 bis 300 nm, Glaspulver mit einer Partikelgröße von 0,2 bis 1,5 µm, insbesondere röntgenopake Glaspulver z.B. von Barium- oder Strontiumaluminiumsilikatgläsern, und röntgenopake Füllstoffe mit einer Partikelgröße von 0,2 bis 5 µm, insbesondere Ytterbiumtrifluorid und/oder Mischoxide von $SiO_2$ mit Ytterbium(III)-oxid.

Zur Verbesserung des Verbundes zwischen den Füllstoffpartikeln und der vernetzten Polymerisationsmatrix können $SiO_2$-basierende Füllstoffe mit methacrylatfunktionalisierten Silanen oberflächenmodifiziert werden. Ein bevorzugtes Beispiel für solche Silane ist 3-Methacryloyloxypropyltrimethoxysilan. Zur Oberflächenmodifizierung von nichtsilikatischen Füllstoffen wie $ZrO_2$ oder $TiO_2$ können auch funktionalisierte saure Phosphate, wie z.B. 10-Methacryloyloxydecyldihydrogenphosphat eingesetzt werden.

Weitere bevorzugte Füllstoffe sind partikuläre Wachse, insbesondere Carnaubawachs, vorzugsweise mit eine Partikelgröße von 1 bis 10 µm, unvernetzte oder teilvernetzte Polymethylmethacrylat (PMMA)-Partikel, vorzugsweise mit einer Partikelgröße von 500 nm bis 10 µm, sowie Polyamid-12 Partikel, vorzugsweise mit einer Partikelgröße von 5 bis 10 µm.

Außerdem können die erfindungsgemäßen Dentalwerkstoffe einen sog. Präpolymerfüllstoff oder Isofüllstoff enthalten, d.h. ein gemahlenes Komposit, das vorzugsweise eine breite Partikelgrößenverteilung z. B. mit Partikelgrößen von 0,05 bis 20 µm, insbesondere etwa 0,1 bis etwa 10 µm, aufweist. Vorzugsweise ist der Präpolymerfüllstoff oder Isofüllstoff oberflächenmodifiziert, insbesondere silanisiert.

Wenn nicht anders angegeben, handelt es sich bei allen Partikelgrößen hierin um gewichtsmittlere Partikelgrößen, wobei die Partikelgrößenbestimmung im Bereich von 0,1 µm bis 1000 µm mittels statischer Lichtstreuung erfolgt, vorzugsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320.

Partikelgrößen kleiner als 0,1 µm werden vorzugsweise mittels Dynamischer Lichtstreuung (DLS) bestimmt. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem Malvern Zetasizer Nano ZS (Malvern Instruments, Malvern UK) mit einem He-Ne-Laser mit einer Wellenlänge von 633 nm, bei einem Streuwinkel von 90° bei 25°C.

Die Lichtstreuung nimmt mit abnehmender Partikelgröße ab. Partikelgrößen kleiner als 0,1 µm können auch mittels REM- oder TEM-Spektroskopie bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 Å dickem Kupfergitter (Maschenweite 300) aufgebracht, das mit Kohlenstoff beschichtet ist, und im Anschluss das Lösungsmittel verdampft.

Die Füllstoffe werden nach ihrer Partikelgröße unterteilt in Makrofüller und Mikrofüller, wobei Füllstoffe mit einer mittleren Partikelgröße von 0,2 bis 10 μm als Makrofüller und Füllstoffe mit einer mittlere Partikelgröße von ca. 5 bis 100 nm als Mikrofüller bezeichnet werden. Makrofüller werden z.B. durch Mahlen z.B. von Quarz, röntgenopaken Gläsern, Borosilikaten oder von Keramik gewonnen und bestehen meist aus splitterförmigen Teilen. Als Mikrofüller werden vorzugsweise pyrogenes $SiO_2$ oder Fällungskieselsäure eingesetzt, oder Mischoxide, z.B. $SiO_2$-$ZrO_2$, die durch hydrolytische Cokondensation von Metallalkoxiden zugänglich sind. Die Mikrofüller haben vorzugsweise eine mittlere Partikelgröße von ca. 5 bis 100 nm. Mikrofüller. Füllstoffe mit geringer Partikelgröße haben eine größere Verdickungswirkung.

In einer bevorzugten Ausführungsform enthalten die erfindungsgemäßen Dentalwerkstoffe eine Mischung von zwei oder mehreren Füllstoffen, insbesondere von zwei oder mehreren Füllstoffen mit unterschiedlichen Partikelgrößen. Es wurde gefunden, dass durch die Verwendung solcher Füllstoffmischungen die Viskosität der Werkstoffe nicht übermäßig erhöht wird und die Zusammensetzungen daher mit generativen Verfahren, wie z.B. mit Hilfe der Stereolithographie, gut verarbeitbar sind. Der Gesamtgehalt an Füllstoffen liegt vorzugsweise in einem Bereich von 0 bis 20 Gew.-%, besonders bevorzugt von 0 bis 10 Gew.-%.

Die erfindungsgemäßen Dentalwerkstoffe können weiterhin einen oder mehrere **UV-Absorber (h)** enthalten. Der UV-Absorber dient dazu, bei der lichtinduzierten Härtung der erfindungsgemäßen Zusammensetzung die Eindringtiefe des Lichts und damit die Polymerisationstiefe zu verringern. Dies erweist sich insbesondere bei stereolithographischen Anwendungen als vorteilhaft, da bei der Stereolithographie lediglich dünne Schichten gehärtet werden sollen. Durch Verwendung eines UV-Absorbers kann bei stereolithographischen Verfahren die Präzision verbessert werden.

Bevorzugt sind UV-Absorber auf der Basis von Benzotriazol, Benzophenon oder Triazine. Besonders bevorzugte UV-Absorber sind 2,2'-Methylenbis[6-(2H-benzotriazol-2-yl)-4-(1,1,3,3-tetramethylbutyl)phenol], 2,2',4,4'-Tetrahydroxybenzophenon, 2-tert-Butyl-6-(5-chloro-2H-benzotriazol-2-yl)-4-methylphenol (Bumetrizol), 2,2'-Benzol-1,4-diylbis(4h-3,1-benzoxazin-4-on), 2-(4,6-Bis-(2,4-dimethylphenyl)-1,3,5-triazin-2-yl)-5-(octyloxy)-phenol, 2-(2-Hydroxy-5-methylphenyl)benzotriazol, 2-(2-Hydroxyphenyl)benzotriazol, 2-(2H-Benzotriazol-2-yl)-6-dodecyl-4-methylphenol, 2-(2'-Hydroxy-3', 5'-di-t-butylphenyl)-5-chlorobenzotriazole, 2,2'-Dihydroxy-4-methoxybenzophenon und 2,2'-Dihydroxy-4,4'-dimethoxybenzophenon. Weiter bevorzugt sind sogenannte Hindered Amine Light Stabilizers wie Bis(1,2,2,6,6-pentamethyl-4-piperidyl)sebacat, Methyl-1,2,2,6,6-pentamethyl-4-piperidylsebacat, Bis(1-octyloxy-2,2,6,6-tetramethyl-4-piperidyl)sebacat und Bis(1,2, 2,6,6-pentamethyl-4-piperidyl)-[[3,5-bis(1,1-dimethylethyl)-4-hydroxyphenyl]methyl]butylmalonat. Ganz besonders bevorzugte UV-Absorber sind Bumetrizol und 2,2',4,4'-Tetrahydroxybenzophenon.

Der UV-Absorber weist vorzugsweise ein Absorptionsmaximum auf, das der Wellenlänge des zur Härtung eingesetzten Lichts entspricht. Vorteilhaft sind UV-Absorber mit einem Absorptionsmaximum im Bereich von 320 bis 500 nm und vorzugsweise 380 bis 480 nm, wobei UV-Absorber mit einem Absorptionsmaximum unterhalb von 400 nm besonders bevorzugt sind.

UV-Absorber werden ggf. in einer Menge von vorzugsweise 0 bis 1,0 Gew.-%, besonders bevorzugt 0,01 bis 0,5 Gew.-% eingesetzt. Bumetrizol wird vorzugsweise in einer Menge von 0,01 bis 0,2 Gew.-%, besonders bevorzugt 0,02 bis 0,15 Gew.-%, und 2,2',4,4'-Tetrahydroxybenzophenon in einer Menge von 0,01 bis 0,07 Gew.-% verwendet. Alle Angaben beziehen sich auf das Gesamtgewicht des Werkstoffs. Dentalwerkstoffe, die keinen UV-Absorber enthalten, sind bevorzugt.

Die erfindungsgemäßen Dentalwerkstoffe können außerdem einen oder mehrere **optische Aufheller (i)** enthalten. Erfindungsgemäß bevorzugt sind optische Aufheller, die Licht im UV-Bereich absorbieren, d.h. Licht mit einer Wellenlänge unterhalb von 400 nm. Durch die Zugabe eines optischen Aufhellers kann die Eindringtiefe des Lichts und damit die Durchhärtungstiefe verringert und so die Präzision bei stereolithographischen Prozessen erhöht werden. Besonders bevorzugt sind optische Aufheller, die in der Lage sind, das im UV-Bereich absorbierte Licht als Licht mit einer Wellenlänge von 400 bis 450 nm wieder zu emittieren. Solche optischen Aufheller erhöhen die Reaktivität der Werkstoffe, indem sie das aufgenommene kurzwellige Licht aufgrund ihrer Fluoreszenz als längerwelliges Blaulicht abstrahlen und damit zusätzliche Lichtleistung zur Photoinitiierung bereitstellen. Erfindungsgemäß bevorzugte optische Aufheller sind 2,5-Bis(5-tert-butyl-benzoxazol-2-yl)thiophen und Fluoreszenzmittel in Form von Terephthalsäurederivaten, wie z.B. 2,5-Dihydroxyterephthalsäurediethylester oder Diethyl-2,5-dihydroxyterephthalat.

Der oder die optischen Aufheller werden ggf. in einer Menge von vorzugsweise 0 bis 0,1 Gew.-%, besonders bevorzugt 0,001 bis 0,05 Gew.-% und ganz besonders bevorzugt 0,002 bis 0,02 Gew.-% eingesetzt, jeweils bezogen auf das Gesamtgewicht des Werkstoffs. Dentalwerkstoffe, die keinen optischen Aufheller enthalten, sind bevorzugt.

Optische Aufheller können in Kombination mit UV-Absorbern eingesetzt werden. In diesem Fall ist es bevorzugt, dass das Gewichtsverhältnis von UV-Absorber zu optischem Aufheller in einem Bereich von 2:1 bis 50:1, besonders bevorzugt 2:1 bis 30:1 und ganz besonders bevorzugt 2:1 bis 5:1 oder 10:1 bis 25:1 liegt. Bevorzugt sind Kombinationen, die als UV-Absorber 2,2',4,4'-Tetrahydroxybenzophenon oder Bumetrizol und als optischen Aufheller 2,5-Bis(5-tert-butylbenzoxazol-2-yl)thiophen enthalten. Ganz besonders bevorzugt ist die Kombination von 2,2',4,4'-Tetrahydroxybenzophenon und 2,5-Bis(5-tert-butyl-benzoxazol-2-yl)thiophen in einem Gewichtsverhältnis von 2:1 bis 10:1, vorzugsweise 2:1 bis 5:1, oder die Kombination von Bumetrizol und 2,5-Bis(5-tert-butyl-benzoxazol-2-yl)thiophen in einem Gewichtsverhältnis von 5:1 bis 30:1, vorzugsweise 10:1 bis 20:1.

Die erfindungsgemäßen Dentalwerkstoffe können darüber hinaus weitere **Additive** (j) enthalten, vor allem Stabilisatoren, Farbmittel, Weichmacher, Thixotropieadditive, mikrobiozide Wirkstoffe und/oder Treibmittel.

Die erfindungsgemäßen Dentalwerkstoffe enthalten vorzugsweise einen oder mehrere **Stabilisatoren.** Hierbei handelt es sich um radikalfangende Stoffe zur Verhinderung einer vorzeitigen Polyreaktion. Die Stabilisatoren werden auch als Polymerisationsinhibitoren bezeichnet. Die Inhibitoren bzw. Stabilisatoren verbessern die Lagerstabilität der Materialien.

Bevorzugte Inhibitoren sind Phenole, wie Hydrochinonmonomethylether (MEHQ) oder 2,6-Di-tert.-butyl-4-methyl-phenol (BHT). Phenole werden vorzugsweise in einer Konzentration von 0,001 bis 0,50 Gew.-% verwendet. Weitere bevorzugte Inhibitoren sind Phenothiazin, das 2,2-Diphenyl-1-picrylhydrazyl (DPPH)-, das Galvinoxyl-, das Triphenylmethyl- und das 2,2,6,6-Tetramethyl-piperindin-1-oxyl-Radikal (TEMPO). Diese Inhibitoren werden vorzugsweise in einer Menge von 0,001 bis 0,02 Gew.-% eingesetzt. Eine Polymerisation findet erst dann statt, wenn diese Additive verbraucht sind. Die Mengenangaben beziehen sich jeweils auf die Gesamtmasse des Materials. Vorzugsweise wird eine Mischung von Inhibitoren eingesetzt, die mindestens ein Phenol und mindestens einen der weiteren Initiatoren enthält.

Darüber hinaus können die erfindungsgemäßen Dentalwerkstoffe auch **Farbmittel** enthalten, vorzugsweise in einer Konzentration von 0,0001 bis 0,5 Gew.-%. Die Farbmittel dienen primär ästhetischen Zwecken. Erfindungsgemäß bevorzugte Farbmittel sind organische Farbstoffe und Pigmente, insbesondere Azofarbstoffe, Carbonylfarbstoffe, Cyaninfarbstoffe, Azomethine und Methine, Phthalocyanine und Dioxazine. Besonders bevorzugt sind Farbstoffe, die in den erfindungsgemäßen Werkstoffen löslich sind, insbesondere Azofarbstoffe. Als Farbmittel eignen sich außerdem anorganische und insbesondere organische Pigmente, die sich gut in den erfindungsgemäßen Dentalwerkstoffe dispergieren lassen. Bevorzugte anorganische Pigmente sind Metalloxide oder Hydroxide, wie z.B. Titandioxid oder ZnO als Weisspigmente, Eisenoxid ($Fe_2O_3$) als Rotpigment oder Eisenhydroxid (FeOOH) als Gelbpigment. Bevorzugte organische Pigmente sind Azopigmente, wie z.B. Monoazogelb- und Orangepigmente, Disazopigmente oder β-Naphthol-Pigmente, und Nichtazo- oder polycyclische Pigmente, wie z.B. Phthalocyanin-, Chinacridon-, Perylen- und Flavanthron-Pigmente. Besonders bevorzugt sind Azopigmente und Nichtazopigmente.

Außerdem können die erfindungsgemäßen Dentalwerkstoffe einen oder mehrere **Weichmacher** enthalten. Weichmacher verhindern, dass die Polymerisate nach der photochemischen Aushärtung und einem evtl. Trocknen brüchig werden. Darüber hinaus sorgen Weichmacher für eine ausreichende Flexibilität. Weichmacher werden vorzugsweise in einer Konzentration von 0,2 bis 5 Gew.-% zugesetzt. Bevorzugte Weichmacher sind Phthalate, wie z.B. Dibutyl- oder Dihexylphthalat, nichtsaure Phosphate, wie z.B. Tributyl- oder Trikresylphosphat, n-Octanol, Glycerin oder Polyethylenglykole. Besonders bevorzugt sind Weinsäure- oder Citronsäureester, wie z.B. Citronensäuretriester, die sich durch eine gute Biokompatibilität auszeichnen.

Die erfindungsgemäßen Dentalwerkstoffe können weiterhin ein oder mehrere **Thixotropieadditive** enthalten. Diese Additive bewirken eine Verdickung der Werkstoffe und können so beispielsweise ein Sedimentieren der Füllstoffe verhindern. Insbesondere füllstoffhaltige Werkstoffe enthalten daher vorzugweise mindestens ein Thixotropieadditiv. Bevorzugte Thixotropieadditive sind OH-Gruppen-haltige Polymere, wie z.B. Cellulosederivate, und anorganische Stoffe, wie z.B. Schichtsilicate. Um die Viskosität der Werkstoffe nicht zu stark zu erhöhen, enthalten die erfindungsgemäßen Dentalwerkstoffe vorzugsweise nur 0 bis 3,0 Gew.-%, besonders bevorzugt 0 bis 2,0 Gew.-% und ganz besonders bevorzugt 0,1 bis 2,0 Gew.-% Thixotropieadditiv, bezogen auf das Gesamtgewicht des Werkstoffs.

Bestimmte Füllstoffe, wie z.B. hochdisperses $SiO_2$, d.h. $SiO_2$ mit geringer Primärteilchengröße (< 20 nm) und hoher Oberfläche (> 100 $m^2$) haben ebenfalls eine thixotropierende Wirkung. Solche Füllstoffe können Thixotropieadditive ersetzen.

Die rheologischen Eigenschaften der erfindungsgemäßen Dentalwerkstoffe werden an den gewünschten Anwendungszweck angepasst. Materialen zur stereolithographischen Verarbeitung werden vorzugsweise so eingestellt, dass ihre Viskosität im Bereich von 50 mPa·s bis 100 Pa·s, bevorzugt 100 mPa·s bis 10 Pa·s, besonders bevorzugt 100 mPa·s bis 5 Pa·s liegt. Die Viskosität wird bei 25°C mit einem Kegel-Platte-Viskosimeter bestimmt (Scherrate 100/s). Besonders bevorzugt weisen die erfindungsgemäßen Dentalwerkstoffe eine Viskosität < 10 Pa·s und ganz besonders bevorzugt < 5 Pa·s bei 25°C auf. Die Viskosität wird vorzugsweise mit einem Anton Paar Viskosimeter des Typs MCR 302 mit CP25-2 Konus-Platte-Messmittel und einem Messspalt von 53 μm in Rotation bei einer Scherrate von 100/s bestimmt. Aufgrund der geringen Viskosität sind die erfindungsgemäßen Dentalwerkstoffe besonders dazu geeignet, mit Hilfe von generativen Fertigungsverfahren, wie z.B. 3D-Druck oder Stereolithographie, verarbeitet zu werden. Die Verarbeitungstemperatur liegt vorzugsweise in einem Bereich von 10 bis 70°C, besonders bevorzugt 20 bis 30°C.

Erfindungsgemäß sind Dentalwerkstoffe mit der folgenden Zusammensetzung besonders bevorzugt:

(a) 30 bis 70 Gew.-%, bevorzugt 30 bis 61 Gew.-% besonders bevorzugt 40 bis 60 Gew.-% mindestens eines aromatischen, bicyclischen oder tricyclischen Mono(meth)acrylats,

(b) 20 bis 60 Gew.-%, bevorzugt 30 bis 55 Gew.-% und besonders bevorzugt 33 bis 55 Gew.-% mindestens eines

Urethandi(meth)acrylat-Telechels mit einer zahlenmittleren Molmasse von 750 bis 2000 g/mol,
(c) 0 bis 30 Gew.-%, bevorzugt 0 bis 20 Gew.-%, und besonders bevorzugt 0 Gew.-% Di(meth)acrylatmonomer(e),
(d) 1 bis 12 Gew.-%, bevorzugt 2 bis 12 Gew.-% besonders bevorzugt 2 bis 10 Gew.-% mindestens eines ABA- bzw. AB-Blockcopolymers,
(e) 0,1 bis 5,0 Gew.-%, bevorzugt 0,2 bis 4,0 Gew.-% und besonders bevorzugt 0,3 bis 3,0 Gew.-% mindestens eines Initiators für die radikalische Polymerisation,
(f) 0 bis 15 Gew.-%, bevorzugt 0 bis 5 Gew.-% und besonders bevorzugt 0 Gew.-% Kern-Schale-Polymerpartikel,
(g) 0 bis 20 Gew.-%, vorzugsweise 0 bis 15 Gew.-% und besonders bevorzugt 0 bis 10 Gew.-% Füllstoff,
(h) 0 bis 1,0 Gew.-%, bevorzugt 0 bis 0,7 Gew.-% und besonders bevorzugt 0 bis 0,5 Gew.-% UV-Absorber,
(i) 0 bis 0,5 Gew.-%, bevorzugt 0 bis 0,1 Gew.-% und besonders bevorzugt 0 bis 0,05 Gew.-% optischen Aufheller und
(j) 0 bis 15 Gew.-%, bevorzugt 0 bis 10 Gew.-% und besonders bevorzugt 0,05 bis 5 Gew.-% an weiteren Additiven.

Ganz besonders bevorzugt sind Dentalwerkstoffe mit der folgenden Zusammensetzung:

(a) 30 bis 70 Gew.-%, bevorzugt 30 bis 61 Gew.-% und besonders bevorzugt 40 bis 60 Gew.-% mindestens eines Mono(meth)acrylats der Formel (I),
(b) 20 bis 60 Gew.-%, bevorzugt 30 bis 55 Gew.-% und besonders bevorzugt 33 bis 55 Gew.-% mindestens eines Urethandi[meth]acrylat-Telechels der Formel (II),
(c) 0 bis 30 Gew.-%, bevorzugt 0 bis 20 Gew.-% und besonders bevorzugt 0 Gew.-% weitere Di(meth)acrylatmonomere,
(d) 2 bis 12 Gew.-%, bevorzugt 2 bis 10 Gew.-% und besonders bevorzugt 3 bis 8 Gew.-% mindestens eines ABA- oder AB-Blockcopolymers,
(e) 0,1 bis 5,0 Gew.-%, bevorzugt 0,2 bis 4 Gew.-% und besonders bevorzugt 0,3 bis 3,0 Gew.-% mindestens eines Photoinitiators,
(f) 0 bis 15 Gew.-%, bevorzugt 0 bis 5 Gew.-% und besonders bevorzugt 0 Gew.-% Kern-Schale-Polymerpartikel,
(g) 0 bis 20 Gew.-%, vorzugsweise 0 bis 15 Gew.-% und besonders bevorzugt 0 bis 10 Gew.-% Füllstoff,
(h) 0 bis 1,0 Gew.-%, bevorzugt 0 bis 0,7 und besonders bevorzugt 0 bis 0,5 Gew.-% UV-Absorber,
(i) 0 bis 0,5 Gew.-%, bevorzugt 0 bis 0,1 Gew.-% und besonders bevorzugt 0 bis 0,05 Gew.-% optischen Aufheller und
(j) 0 bis 15 Gew.-%, bevorzugt 0 bis 10 Gew.-% und besonders bevorzugt 0,02 bis 5 Gew.-% an weiteren Additiven.

Wenn nicht anders angegeben beziehen sich alle Gewichtsprozentangaben hierin auf die Gesamtmasse des Dentalwerkstoffs.
Erfindungsgemäß besonders bevorzugt sind Dentalwerkstoffe, die

(a) 40 bis 61 Gew.-% an 2-Phenoxyethyl(meth)acrylat, 2-(o-Biphenyloxy)ethyl-(meth)acrylat, 2-Hydroxy-3-phenoxy-propyl-(meth)acrylat, 2-[(Benzyloxycarbonyl)amino]ethyl(meth)acrylat, 1-Phenoxypropan-2yl-(meth)acrylat, 2-(Ben-zyloxy)-ethyl(meth)acrylat, 2-(Methaacryloyloxy)ethyl(meth)acrylat, 3-Phenoxy-benzyl(meth)acrylat, Phenoxypropyl(meth)acrylat, 2-Benzyloxyethyl(meth)-acrylat, 2-Benzoyloxyethyl(meth)acrylat, 2-(Meth)acryloyloxybenzoesäure-methylester, 2-Phenylethyl(meth)acrylat, Tricyclodecan(meth)acrylat, Tricyclo-decanmethyl(meth)acrylat und/oder 2-(p-Cumylphenoxy)ethylmethacrylat,

(b) 33 bis 55 Gew.-% mindestens eines Urethandi(meth)acrylat-Telechels mit einer zahlenmittleren Molmasse von 750-2000 g/mol und mit mindestens 4 Urethangruppen, hergestellt durch Reaktion von 1 Mol an ethoxyliertem oder propoxyliertem Bisphenol-A, Decan- oder Dodecandiol mit 2 Mol Isophorondiisocyanat (IPDA) und anschließender Umsetzung mit 2 Mol 2-Hydroxyethylmethacrylat (HEMA) oder Hydroxypropylmethacrylat (HPMA),

(c) 0 Gew.-% weitere Di(meth)acrylatmonomere,

(d) 2 bis 10 Gew.-% mindestens eines ABA- oder AB-Blockcopolymers, wobei der A-Block aus oligomeren Polycaprolacton-, Poly(2,6-dimethyl-1,4-phenylenoxid)-, Poly(ethylenoxid)-, Poly(propylenoxid)- oder Poly(meth)-acrylat-Bausteinen und der B-Block aus Poly(dimethylsiloxan)-, Poly(isopren)-, Poly(vinylacetat)-, Poly(isobuten)-, cis-Poly(butadien)- oder Poly(ethylen)-Bausteinen aufgebaut ist,

(e) 0 % Kern-Schale-Polymere,

(f) 0,1 bis 5,0 Gew.-% mindestens eines Photoinitiators und

(g) 0,2 bis 5 Gew.-% eines oder mehrerer weitere Additiven enthalten.

Die erfindungsgemäßen Dentalwerkstoffe zeichnen sich dadurch aus, dass sie eine hohe Bruchzähigkeit und Brucharbeit und gleichzeitig eine gute Biegefestigkeit und ein relativ hohes E-Modul aufweisen, gemessen bei 37°C in Wasser, was oralen Bedingungen entspricht. Die Werkstoffe haben außerdem eine hohe Transparenz und eine niedrige Viskosität. Besonders vorteilhaft ist, dass die Dentalwerkstoffe auch nach der Härtung noch eine hohe Transparenz und eine geringe Eigenfarbe haben.

Die im Stand der Technik als Schlagzähmodifikatoren eingesetzten Kern-Schale-Polymere bewirken im Gegensatz dazu in der Regel eine mehr oder weniger ausgeprägte Verringerung der Transparenz, was für generative Verfahren nachteilig ist. Es wurde gefunden, dass die Blockcopolymere (d) eine Verbesserung der Bruchzähigkeit ermöglichen, im Vergleich zu Kern-Schale-Polymere aber nur eine relativ geringe Beeinträchtigung der Transparenz zur Folge haben. Da im Vergleich zu Kern-Schale-Polymeren zur Erzielung der gewünschten Bruchzähigkeit außerdem eine deutlich geringere Menge an Blockcopolymeren (d) notwendig ist, ermöglichen die Blockcopolymere (d) die Herstellung von Werkstoffen mit hoher Transparenz, die sich hervorragend für generative Verfahren eignen.

Die bruchzähigkeitssteigernde Wirkung der Blockpolymeren (d) ist dann besonders ausgeprägt, wenn die Vernetzungsdichte $v_c$ der Materialien in einem Bereich von 300 bis 5000 mol/m$^3$ und vorzugsweise 400 bis 3000 mol/m$^3$ liegt. Eine höhere Vernetzungsdichte bewirkt zwar eine Erhöhung der Biegefestigkeit und des E-Moduls, führt jedoch zu einer geringeren Bruchfestigkeit der Polymerisate. Eine Verringerung der Vernetzungsdichte erhöht die Bruchzähigkeit, wirkt sich aber nachteilig auf die Biegefestigkeit und den E-Modul aus.

Eine Vernetzungsdichte im bevorzugten Bereich wird durch die Verwendung von Urethandi(meth)acrylat-Telechelen (b) mit einer zahlenmittleren Molmasse von 750 bis 2000 g/mol als Vernetzer erreicht, wobei die Vernetzungsdichte durch die Zugabe geringer Mengen an monomeren Di(meth)acrylaten feinjustiert werden kann.

Erfindungsgemäß sind Werkstoffe mit einer Transparenz $\geq$ 60 %, vorzugsweise $\geq$ 70% und ganz besonders bevorzugt $\geq$ 80%, und einer Viskosität $\leq$ 10,0 Pa·s, vorzugsweise $\leq$ 5,0 Pa·s, besonders bevorzugt. Die Transparenz wird wie oben beschreiben gemäß der Norm ISO 10526:1999 gemessen. Die Viskosität wird auf die oben beschriebene Weise mit einem Kegel-Platte-Viskosimeter bestimmt.

Die erfindungsgemäßen Werkstoffe weisen nach der Härtung eine Bruchzähigkeit $K_{max}$ von größer 1,1 MPa·m$^{1/2}$, bevorzugt größer 1,2 MPa·m$^{1/2}$, besonders bevorzugt größer 1,4 MPa·m$^{1/2}$ sowie eine Brucharbeit FW größer 250 J/m$^2$, bevorzugt größer 300 J/m$^2$, besonders bevorzugt größer 400 J/m$^2$ auf. Werkstücke, die aus diesen Materialien hergestellt werden, halten somit in hohem Maße Verformungen ohne Bruch stand. Eine hohe Transparenz in Kombination mit einer hohen Brucharbeit lassen sich mit Kern-Schale-Polymeren nicht erreichen.

Die Bestimmung der Bruchzähigkeit $K_{max}$ und der Brucharbeit FW erfolgt in Anlehnung an ISO 20795-1:2013 im 3-Punkt Biegeversuch bei einer Stützweite von 32 mm. Die Bestimmung von $K_{max}$ und FW basieren dabei auf den theoretischen Grundlagen des Spannungsintensitätsfaktors $K_{1C}$. Die Bruchzähigkeit $K_{max}$ ist der Höchstfaktor der Beanspruchungsintensität, die auch als Spannungsintensitätsfaktor bei Höchstlast bezeichnet wird, und wird wie folgt berechnet:

$$K_{max} = \left( \frac{P_{max} \cdot S}{B \cdot W^{3/2}} \right) \cdot f(x) \cdot 0.031 \, \text{MPa} \cdot \text{m}^{1/2},$$

mit

$$f(x) = 3 \cdot \sqrt{x} \, \frac{1.99 - x(1-x) \cdot (2.15 - 3.93x + 2.7x^2)}{2(1+2x) \cdot (1-x)^{3/2}},$$

wobei

$$x = \frac{a}{W}$$

und W die Probenkörperhöhe (= 8 mm), B die Probenkörperdicke (= 4 mm), a die Anrisslänge (= 3 mm + Anrisstiefe mit Rasierklinge), S die Stützweite (= 32 mm) und $P_{max}$ der maximale Druck bei der Prüfung ist.

Die Brucharbeit FW (fracture work, gesamte Brucharbeit) wird auf folgende Weise berechnet:

$$FW = \frac{U}{2B(W-a)} \cdot 1000\ \text{J/m}^2$$

wobei U die gesamte Energie ist, die für den Bruch der Probe nötig ist (Integral der Last/Weg Grafik) und die gebraucht wird, um die zwei neuen Bruchflächen $B(W-a)$ zu erzeugen. Dieser Parameter beschreibt den Widerstand des Materials gegen die Rissausbreitung.

Die erfindungsgemäßen Werkstoffe weisen nach der Härtung eine gute Biegefestigkeit und ein relativ gutes Biegemodul sowie eine gute Bruchzähigkeit und eine hohe Brucharbeit auf. Formteile, die durch Härtung der erfindungsgemäßen Werkstoffe erhalten werden, haben eine hohe Steifigkeit und setzen einer Verformung einen hohen Widerstand entgegen, ohne zu brechen. Bevorzugt sind Materialien, die nach der Härtung eine Biegefestigkeit, bestimmt gemäß ISO20795-1:2013, von mindestens 40 MPa, besonders bevorzugt 50 MPa oder mehr und ganz besonders bevorzugt von 60 MPa oder mehr haben. Außerdem weisen die gehärteten Materialen vorzugsweise ein Biegemodul, bestimmt gemäß ISO20795-1:2013, von mindestens 1000 MPa, bevorzugt von 1300 MPa oder mehr, besonders bevorzugt von 1500 MPa oder mehr, ganz besonders bevorzugt 2000 MPa oder mehr und am meisten bevorzugt von 2500 MPa auf. Zudem haben die Werkstoffe eine Bruchzähigkeit $K_{max}$ von 1,1 $MPa \cdot m^{1/2}$ oder größer, bevorzugt 1,2 $MPa \cdot m^{1/2}$ oder größer, besonders bevorzugt 1,4 $MPa \cdot m^{1/2}$ oder größer sowie eine Brucharbeit FW von 250 $J/m^2$ oder größer, bevorzugt 300 $J/m^2$ oder größer, besonders bevorzugt 400 $J/m^2$ oder größer. Besonders bevorzugt sind demnach Werkstoffe mit einer Biegefestigkeit von 60 bis 100 MPa, einem Biegemodul von 2000 bis 2500 MPa, einer Bruchzähigkeit $K_{max}$ von 1,4 bis 2,5 $MPa \cdot m^{1/2}$ und einer Brucharbeit FW von 400 bis 800 $J/m^2$.

Gemäß einer besonders bevorzugten Ausführungsform der Erfindung haben die Werkstoffe nach der Härtung eine Biegefestigkeit von 50 MPa oder größer, gemessen nach ISO220795-1:2013, und eine Brucharbeit 250 $J/m^2$ oder größer und einen $K_{max}$-Wert (Höchstfaktor der Beanspruchungsintensität) von mindestens 1,2 $MPa \cdot m^{0,5}$.

Aufgrund der obigen Eigenschaften eignen sich die erfindungsgemäßen Werkstoffe hervorragend zur Verwendung als Dentalwerkstoff, z.B. als Prothesenwerkstoff oder Verblendmaterial, und insbesondere zur Herstellung oder Reparatur von dentalen Formteilen, wie z.B. Dentalrestaurationen, Prothesen, künstlichen Zähnen, Inlays, Onlays, Kronen, Brücken, Bohrschablonen, Splints (Aufbissschienen), Einprobekörpern und orthodontischen Vorrichtungen, wie z.B. Kunststoffkorrekturschienen, sog. Alignern und Positionierern. Die genannten Formteile sind ebenfalls Gegenstand der Erfindung. Die erfindungsgemäßen Dentalwerkstoffe werden vorzugsweise extraoral, d.h. nicht therapeutisch verwendet.

Ein weiterer Gegenstand der vorliegenden Erfindung ist ein Verfahren zur Herstellung von dentalen Formteilen, insbesondere zur Herstellung der oben genannten dentalen Fortteile, bei dem eine erfindungsgemäße Zusammensetzung mit Hilfe von Licht gehärtet wird, um das dentale Formteil zu ergeben. Die Herstellung oder Reparatur von dentalen Formteilen erfolgt vorzugsweise extraoral, besonders bevorzugt durch ein generatives Verfahren, ganz besonders bevorzugt durch 3D-Druck oder ein Lithographie-basiertes Verfahren, wie z.B. die Stereolithographie.

Die stereolithographische Herstellung von Formteilen erfolgt vorzugsweise indem durch die direkte oder indirekte Digitalisierung des zu restaurierenden Zahns bzw. der zu restaurierenden Zähne am Computer ein virtuelles Abbild der Zahnsituation erstellt wird, dann anhand dieses Abbilds am Computer ein Modell der Dentalrestauration oder Prothese konstruiert wird und dieses anschließend durch generative stereolithographische Fertigung hergestellt wird.

Nach dem Erstellen des virtuellen Modells des herzustellenden dentalen Werkstückes wird die erfindungsgemäße Zusammensetzung durch selektive Lichteinstrahlung polymerisiert. Die Dentalrestauration oder Prothese wird vorzugsweise schichtweise aufgebaut, indem nacheinander eine Vielzahl von dünnen Schichten mit dem gewünschten Querschnitt polymerisiert wird. Nach dem schichtweisen Aufbau der Restauration oder Prothese wird vorzugsweise überschüssiges Restharz entfernt. Dies kann durch geeignete mechanische Verfahren (z.B. Schleudern oder Abblasen) oder durch Behandlung mit einem geeigneten Lösungsmittel, wie z.B. einem Alkohol, wie Ethanol oder Isopropanol, einem Keton, wie z.B. Aceton oder einem Ester, wie z.B. Ethylacetat, erfolgen. Anschließend erfolgt vorzugsweise eine Nachvergütung durch Erwärmen oder besonders bevorzugt durch Bestrahlung des Werkstücks mit Licht einer geeigneten Wellenlänge, wie z.B. die Bestrahlung mit Licht einer Intensität von z.B. 160 $mW/cm^2$ bei 405 nm. Bei der Verwendung von zwei Photoinitiatoren ist die Bestrahlung mit zwei unterschiedlichen Wellenlängen vorteilhaft. Vorzugsweise wird das Werkstück gleichzeitig oder in einem nachfolgenden Schritt auf eine Temperatur oberhalb von 50 °C erwärmt. Durch die photochemische und/oder thermische Nachvergütung lassen sich die mechanischen Eigenschaften verbessern.

Die Erfindung wird im Folgenden anhand von Beispielen näher erläutert.

**Ausführungsbeispiele**

**Beispiel 1**

<u>*Synthese von 2-(2-Biphenyloxy)-ethylmethacrylat (aromatisches Monomethacrylat)*</u>

*1. Stufe: 2-(2-Biphenyl)-oxyethanol*

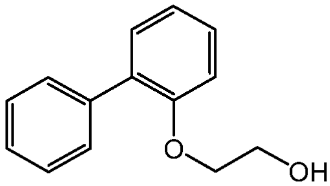

In einem Doppelmantelreaktor wurden zu einer Lösung von 0,90 kg (22,5 mol) Natriumhydroxid in 15,0 kg Wasser 2,55 kg (15,0 mol) 2-Phenylphenol, 0,12 kg (0,75 mol) Kaliumiodid und 0,17 kg (0,75 mol) Benzyltriethylammoniumchlorid zugegeben. Die Lösung wurde auf 60 °C (Innentemperatur) erwärmt und gleichzeitig mit dem Zutropfen von 1,81 kg (22,5 mol) 2-Chlorethanol begonnen. Nach beendeter Zugabe wurde der Ansatz für 48 h bei 60 °C gerührt. Zur Aufarbeitung wurde der Ansatz mit 6,0 L Toluol verdünnt, und nach der Phasentrennung wurde die wässrige Phase noch 2-mal mit je 3,0 L Toluol extrahiert. Die vereinten Toluolphasen wurden 3-mal mit je 4,0 L 1N Natronlauge, 3-mal mit je 4,0 L 1N Salzsäure sowie 3-mal mit je 3,0 L Wasser gewaschen. Das Toluol wurde im Vakuum abdestilliert. Nach einer Umkristallisation aus Toluol erhielt man 2,77 kg (86 % Ausbeute) 2-(2-Biphenyl)-oxyethanol als farblosen, kristallinen Feststoff (Schmp.: 74-75 °C) mit einer Reinheit von >99 % (GC).

$^1$H-NMR (400 MHz, $CDCl_3$) $\delta$ (ppm) = 1,86 (t, J = 6,5 Hz, 1H, OH), 3,78-3,81 (m, 2H, $HOCH_2$), 4,04 (t, J = 4,6 Hz, 2H, $OCH_2$), 6,98-7,08, 7,28-7,34, 7,38-7,42 und 7,50-7,52 (4 m, 2H, 3H, 2H, 2H, =CH).

$^{13}$C-NMR (100 MHz, $CDCl_3$) $\delta$ (ppm) = 61,4 und 70,3 ($OCH_2$), 113,5 (C-6), 121,7 (C-4), 127,1, 128,7 und 131,0 (C-3, C-5, C-4'), 128,1 und 129,4 (C-2', C-3', C-5', C-6'), 131,5 und 138,4 (C-2, C-1'), 155,4 (C-1).

IR (Diamant-ATR): ν ($cm^{-1}$) = 3329 (br, m, OH), 3056 (m, =CH), 2918 und 2866 (m, $CH_2$), 1596 und 1584 (m, C=C), 1502 und 1483 (s, Aromat), 1431 (s, $CH_2$), 1260 und 1077 (s, COC), 1054 (s, COH), 749, 730 und 700 (vs, =CH).

*2. Stufe: 2-(2-Biphenyloxy)-ethylmethacrylat*

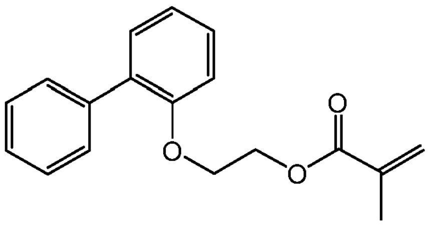

In einem Doppelmantelreaktor wurden zu einer Lösung von 1,33 kg (6,2 mol) 2-(2-Biphenyl)-oxyethanol in 13,0 L Methylenchlorid 0,75 kg (7,4 mol) Triethylamin, 37,9 g (0,31 mol) 4-Dimethylaminopyridin und 0,35 g 2,6-Di-tert-butyl-4-methylphenol zugegeben. Bei 0 °C (Innentemperatur) wurde eine Lösung von 1,14 kg (7,4 mol) Methacrylsäureanhydrid zugetropft und noch 2 St. bei dieser Temperatur sowie 20 h bei 20 °C gerührt. Anschliessend wurde die Lösung 3-mal mit je 4,0 L 1N Salzsäure, 3-mal mit je 4,0 L 1 N Natronlauge sowie 3-mal mit je 4,0 L Wasser gewaschen. Die organische Phase wurde mit 0,09 g Phenothiazin stabilisiert. Nach der Entfernung des Lösungsmittels erhielt man 1,71 kg (98 % Ausbeute) 2-(2-Biphenyloxy)-ethylmethacrylat (BPOEMA) als fast farbloses Öl mit einer Reinheit von 96,45 % (GC).

$^1$H-NMR (400 MHz, $CDCl_3$) $\delta$ (ppm) = 1,93 (s, 3H, $CH_3$), 4,19 und 4,41 (2 t, je J = 4,8 Hz, je 2H, $OCH_2$), 5,55 und 6,08 (2 s, je 1H, $=CH_2$), 6,95-7,06, 7,26-7,30, 7,33-7,37 und 7,52-7,55 (4 m, 2H, 2H, 3H, 2H, =CH).

$^{13}$C-NMR (100 MHz, $CDCl_3$) $\delta$ (ppm) = 18,3 ($CH_3$), 63,0 und 66,5 ($OCH_2$), 113,1 (C-6), 121,7 (C-4), 126,0 ($C=CH_2$), 126,9, 128,6, 131,1 (C-3, C-5, C-4'), 127,9 und 129,6 (C-2', C-3', C-5', C-6'), 131,3 und 138,3 (C-2, C-1'), 136,1 ($C=CH_2$), 155,4 (C-1), 167,2 (C=O).

IR (Diamant-ATR): ν ($cm^{-1}$) = 3027 (w, =CH), 2955 und 2900 (w, $CH_2$, $CH_3$), 1716 (vs, C=O), 1636 (m,

$C{=}C_{Methacryl}$), 1598 und 1584 (m, $C{=}C_{Aromat}$), 1504 und 1482 (m, s, Aromat), 1434 (s, $CH_2$, $CH_3$), 1261 und 1125 (s, $COC_{Ether}$), 1157 (vs, $COC_{Ester}$), 939 (s, $=CH_{Methacryl}$), 751, 733 und 697 (vs, $=CH_{Aromat}$).

Die Rotationsviskosität von BPOEMA wurde mit einem MCR Rheometer (Anton Paar GmbH, Österreich) zu $\eta$ = 0,01 Pa·s bestimmt. Der Brechungsindex von BPOEMA wurde mittels eines Refraktometers ABBE 5 (Bellingham+Stanley, UK) zu $n_D^{20}$ = 1,5729 bestimmt. Die Dichte von BPOEMA wurde mittels eines Biegeschwingers Density Meter DS 7000 (Küss) zu 1,119 g/cm³ bestimmt. Für das Monomer wurde ein Polymerisationsschrumpf von nur 7,4 Vol.-% bestimmt.

**Beispiel 2**

*Synthese von erfindungsgemäßen Urethandimethacrylat-Telechelen*

*Allgemeine Vorschrift zur Umsetzung von Diolen mit Diisocyanaten und HEMA-Endcapping (1:2:2)*

Eine Mischung aus 1 Äquivalenten Diol, 2 Äquivalenten Isophorondiisocyanat (IPDI) und 700 ppm (bezogen auf IPDI) Metatin 712 wurde auf 40 °C erwärmt. Das Diol löste sich dabei komplett auf und das Gemisch erhitzte sich auf ca. 110 °C. Nach Abklingen der Exothermie wurde das Gemisch für 1 h bei 80°C Badtemperatur gerührt, bevor 2 Äquivalenten 2-Hydroxyethylmethacrylat (HEMA, stabilisiert mit 30 ppm (bezogen auf 100 % Produkt) BHT) zugetropft wurden. Nach dem erneuten Abklingen der Exothermie wurden noch 10 min bei 90 °C gerührt. Durch IR- und NMR-Spektroskopie wurde die Vollständigkeit der Reaktion geprüft. Die Addukte wurden als farblose, sehr hochviskose bis spröde Harze erhalten.

*A. 1,10-Decandiol-IPDI-HEMA-Addukt (1:2:2), Isomerengemisch (DMA-Telechel-1, Molmasse: 879,15 g/mol)*

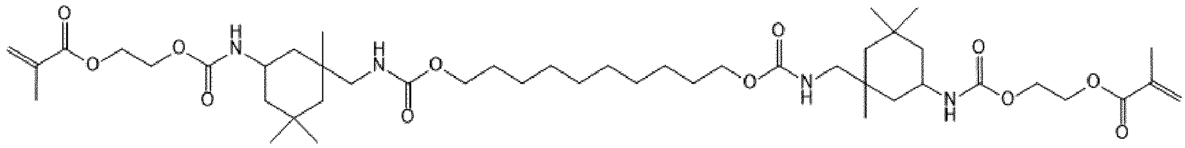

Als Diol wurde 1,10-Decandiol eingesetzt.

$^1H$ NMR (400 MHz, $CDCl_3$): $\delta$ (ppm) = 0,85-0,97, 1,01-1,06, 1,19-1,38 und 1,59-1,75 [4 m, 46H, $(CH_2)_8$, $CH_{2,\ cycl}$, $CH_{3,\ cycl}$), 2,03 (s, 6H, $CH_3$, methacryl), 2,81-2,98 und 3,20-3,33 (2 m, 4H, $NCH_2$), 3,65-3,88 [m, 2H, NCH], 3,98-4,11 (m, 4H, $O\underline{CH_2}(CH_2)_8$), 4,26-4,40 (m, 8H, $O(CH_2)_2O$), 4,57-4,94 (m, 4H, NH), 5,60 und 6,14 (2 s, je 2H, $=CH_2$).

**IR** (diamond ATR): v ($cm^{-1}$) = 3341 (br, NH), 2927 und 2856 (m, C-H), 1695 (vs, C=O), 1638 (m, C=C), 1526 (s, NH), 1456 (m, $CH_2$, $CH_3$), 1366 (m, $CH_3$), 1236 (C-N), 1167 und 1039 (s, m, COC), 942 (m, =CH), 774 [m, $(CH_2)_8$].

*B. Bisphenol A-IPDI-HEMA-Addukt (1:2:2), Isomerengemisch (DMA-Telechel-2, Molmasse: 1049,31 g/mol)*

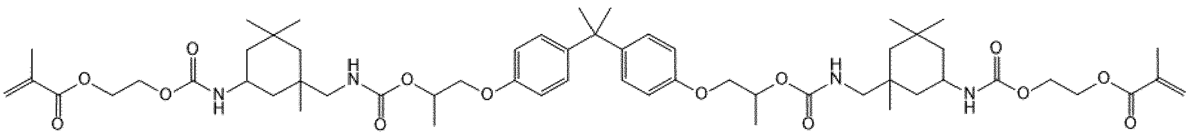

Als Diol wurde ein Bisphenol-A-Derivat mit seitenständigen iso-Propoxy-Gruppen gemäß folgender Formel eingesetzt:

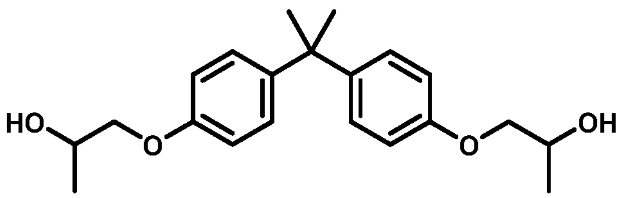

$^1H$ NMR (400 MHz, $CDCl_3$): δ (ppm) = 0,86-1,06, 1,18-1,21, 1,29-1,40 und 1,62-1,75 [4 m, 42H, $CH_{2,\ cycl.}$, $CH_3$), 1,95 (s, 6H, $CH_{3,\ methacryl}$), 2,87-2,99 und 3,19-3,36 (2 m, 4H, $NCH_2$), 3,67-3,88 [m, 2H, NCH], 3,91-4,03 und 4,09-4,22 (2 m, 4H, $OC\underline{H_2}CH$), 4,26-4,40 und 4,50-5,18 (2 m, 14H, $O(CH_2)_2O$, $OCH_2C\underline{H}$, NH), 5,59 und 6,15 (2 s, je 2H, $=CH_2$), 6,80 und 7,12 (2 d, je 4H, =CH).

**IR** (diamond ATR): ν ($cm^{-1}$) = 3341 (br, NH), 2955 und 2860 (m, C-H), 1705 (vs, C=O), 1640 (w, C=C), 1608 (w, Aromat), 1507 (s, NH), 1456 (m, $CH_2$, $CH_3$), 1385 (m, $CH_3$), 1231 (C-N), 1155 und 1043 (s, m, COC), 941 (m, =CH), 829 (m, $=CH_{aromat}$).

**Beispiel 3**

*Synthese von PCL(1600)-b-PDMS(3200)-b-PCL(1600)-Block-Copolymer (PO-277)*

*1. Stufe: Tetramethylammonium-3-aminopropyldimethylsilanoat*

Unter Schutzgasatmosphäre wurde ein Gemisch aus 1,3-Bis(3-aminopropyl)tetramethyldisiloxan (2,49 g, 10,0 mmol) und Tetramethylammoniumhydroxid-Pentahydrat (3,62 g, 20 mmol) in Tetrahydrofuran (THF; 10 ml) 3 h am Rückfluss erhitzt. Das Lösungsmittel wurde abdestilliert und der Rückstand wurde am Feinvakuum auf 50 °C erwärmt. Der gelbliche Rückstand wurde aus THF (20 ml) umkristallisiert. Man erhielt 3,17 g (15,4 mmol; 77 %) eines weißen Feststoffs.

$^1H$-NMR ($CDCl_3$, 400 MHz): δ = 3,16 (s, 12H; $N^+$-$CH_3$), 2,37(t, 2H; J = 7,1 Hz; N-$CH_2$), 1,28 (m, 2H; $CH_2$), 0,14 (m, 2H; Si-$CH_2$), -0,33 (s, 6H; Si-$CH_3$).

*2. Stufe: Polydimethylsiloxan-αω-dipropyl-3-amin: PDMS(3200)*

Unter Schutzgasatmosphäre wurde ein Gemisch aus 1,3-Bis(3-aminopropyl)-tetramethyldisiloxan (4,98 g, 20,0 mmol) und Octamethylcyclotetrasiloxan (12,00 g, 40 mmol) auf 80 °C erwärmt. Tetramethylammonium-3-aminopropyldimethylsilanoat (20 mg) wurde zugegeben und es wurde weiter bei 80 °C gerührt. Nach 30 min wurde mit Argon gesättigtes Octamethylcyclotetrasiloxan (56,00 g, 0,192 mol) langsam zugetropft. Das Reaktionsgemisch wurde weitere 18 h bei 80 °C gerührt und dann zur Zersetzung des Katalysators 30 min auf 150 °C erhitzt. Flüchtige Komponenten wurden anschließend am Feinvakuum entfernt. Man erhielt 65,30 g (88%) eines farblosen Öls.

$^1H$-NMR ($CDCl_3$, 400 MHz): δ =2,64 (t, 4H; J = 7,0 Hz; N-$CH_2$), 1,43 (m, 4H; $CH_2$), 0,51 (m, 4H; Si-$CH_2$), 0,05 (s, 250H; Si-$CH_3$).

*3. Stufe: PCL(1600)-b-PDMS(3200)-b-PCL(1600)-Block-Copolymer*

Ein Gemisch aus PDMS(3200) (20,00 g) und ε-Caprolacton (20,40 g) wurde auf 80 °C erhitzt. Nach 1 h wurde Zinn-bis(2-ethylhexanoat) (10 mg) zugegeben und die Badtemperatur wurde innerhalb von 0 min stufenweise auf 130 °C erhöht. Das nun klare Reaktionsgemisch wurde weitere 5 h bei 130 °C gerührt. Anschließend wurden flüchtige Komponenten am wird am Feinvakuum abdestilliert. Man erhielt 39,50 g (98 %) des Blockcopolymers als einen wachsartigen, leicht gelblichen Feststoff.

$^1H$-NMR ($CDCl_3$, 400 MHz): δ = 3,99 (t, 55H; J = 6,8 Hz; O-$CH_2$), 3,57 (t, 4H; J = 6,8 Hz; HO-$CH_2$), 3,15 (q, 4H; J = 6,8 Hz; N-$CH_2$), 2,24 (t, 55H; J = 7,5 Hz; C(O)-$CH_2$), 2,10 (t, 4H; J = 7,5 Hz; N-$CH_2$), 1,58 (m, 118H; $CH_2$), 1,32 (m, 59H; $CH_2$), 0,46 (m, 4H; Si-$CH_2$), 0,02 (s, 250H; Si-$CH_3$).

**Beispiel 4**

*Synthese von PMMA(1200)-b-PDMS(3200)-b-PMMA(1200)-Block-Copolymer*

*1. Stufe: α, $\overline{\omega}$-(2-Bromoisobutyrylaminopropyl)-poly(dimethylsiloxan)*

Zu einer Lösung von PDMS(3200) (6,66 g; 2,08 mmol) und Triethylamin (0,84 g; 8,44 mmol) in THF (100 ml) gelöst wurde bei 0 °C α-Bromisobuttersäurebromid (1,44 g; 6,24 mmol) zugetropft. Das Reaktionsgemisch wurde 2 h unter Eiskühlung und 18 h bei Umgebungstemperatur gerührt. Die Suspension wurde filtriert und das Filtrat am Rotationsverdampfer eingeengt. Das farblose Öl wurde in Dichlormethan (100 ml) gelöst und mit gesättigter wässriger $Na_2CO_3$-Lösung (2-mal 50 ml), Salzsäure (0,2N, 2-mal 50 ml) und gesättigter wässriger NaCl-Lösung (100 ml) gewaschen, über wasserfreiem Natriumsulfat getrocknet, filtriert und am Rotationsverdampfer eingeengt. Flüchtige Komponenten wurden am Feinvakuum entfernt. Man erhielt 6,32 g (1,81 mmol; 87 %) einer leicht gelblichen Flüssigkeit.

$^1H$-NMR ($CDCl_3$, 400 MHz): δ = 3,22 (m, 4H; N-$CH_2$), 1,92 (s, 12H; C-$CH_3$), 1,54 (m, 4H; $CH_2$), 0,53 (m, 4H; Si-$CH_2$), 0,05 (s, 258H; Si-$CH_3$).

*2. Stufe: PMMA(1200)-b-PDMS(3200)-b-PMMA(1200)-Block-Copolymer*

Unter Inertgasbedingungen wurden $\alpha,\overline{\omega}$-(2-Bromoisobutyrylaminopropyl)-poly(di-methylsiloxan) (6,22 g; 1,78 mmol), Kupfer(I)-chlorid (0,35 g; 3,56 mmol) und N,N,N',N",N"-Pentamethyldiethylentriamin (0,62 g; 3,56 mmol) mit Toluol (50 ml) versetzt und die Lösung wurde entgast. Methacrylsäuremethylester (7,78 g; 77,7 mmol) wurden zugegeben. Die Lösung wurde 30 min bei Umgebungstemperatur gerührt und anschliessend 20 h auf 90 °C erwärmt. Nach dem Abkühlen wurde die Lösung über neutrales Aluminiumoxid filtriert. Das Filtrat wurde am Rotationsverdampfer eingeengt. Der Rückstand wurde in Dichlormethan gelöst und über Kieselgel filtriert. Das Filtrat wurde am Rotationsverdampfer eingeengt und der Rückstand wurde am Feinvakuum getrocknet. Man erhielt 8,77 g (1,49 mmol; 84 %) des Blockcopolymeren als einen gelblichen Feststoff.

$^1$H-NMR ($CDCl_3$, 400 MHz): $\delta$ = 3,53 (s, 72H; O-$CH_3$), 3,28-3,03 (m, 4H; N-$CH_2$), 2,20-1,12 (m, 64H; C-$CH_3$, $CH_2$), 1,13-0,63 (m 72H, $CH_3$), 0,55-0,40 (m, 4H; Si$CH_2$), 0,0 (s, 252H, Si-$CH_3$).

**Beispiel 5**

*Synthese von PCL(2500)-b-PDMS(3200)-b-PCL(2500)-Block-Copolymer*

Ein Gemisch aus PDMS(3200) (20,00g) und $\varepsilon$-Caprolacton (30,60g) wurde auf 80 °C erhitzt. Nach 1 h wurde Zinn-bis(2-ethylhexanoat) (10 mg) zugegeben und die Badtemperatur wurde innerhalb von 30 min stufenweise auf 130 °C erhöht. Das nun klare Reaktionsgemisch wurde weitere 5 h bei 130 °C gerührt. Anschließend wurden flüchtige Komponenten am Feinvakuum abdestilliert. Man erhielt 49,00 g (97 %) eines wachsartigen, leicht gelblichen Feststoffs.

$^1$H-NMR ($CDCl_3$, 400 MHz): $\delta$ = 3,99 (t, 86H; J = 6.8 Hz; O-$CH_2$), 3,57 (t, 4H; J = 6,8 Hz; HO-$CH_2$), 3,15 (q, 4H; J = 6,8 Hz; N-$CH_2$), 2,24 (t, 86H; J = 7.5 Hz; C(O)-$CH_2$), 2,10 (t, 4H; J = 7.5 Hz; N-$CH_2$), 1,60-1,54 (m, 180H; $CH_2$), 1,34-1,29 (m, 90H; $CH_2$), 0,51-0,40 (m, 4H; Si-$CH_2$), 0,02 (s, 250H; Si-$CH_3$).

**Beispiel 6**

*Synthese von PCL(3200)-b-PDMS(3200)-b-PCL(3200)-Block-Copolymer*

Ein Gemisch aus PDMS(3200) (15,00g) und $\varepsilon$-Caprolacton (30,60g) wurde auf 80 °C erhitzt. Nach 1 h wurde Zinn-bis(2-ethylhexanoat) (10 mg) zugegeben und die Badtemperatur wurde innerhalb von 30 min stufenweise auf 130 °C erhöht. Das nun klare Reaktionsgemisch wurde weitere 5 h bei 130 °C gerührt. Anschließend wurden flüchtige Komponenten am Feinvakuum abdestilliert. Man erhielt 44,20 g (97 %) eines wachsartigen, leicht gelblichen Feststoffs.

$^1$H-NMR ($CDCl_3$, 400 MHz): $\delta$ = 3,99 (t, 106H; J = 6,8 Hz; O-$CH_2$), 3,57 (t, 4H; J = 6.8 Hz; HO-$CH_2$), 3,15 (q, 4H; J = 6,8 Hz; N-$CH_2$), 2,24 (t, 106H; J = 7,5 Hz; C(O)-$CH_2$), 2,10 (t, 4H; J = 7,5 Hz; N-$CH_2$), 1,60-1,54 (m, 220H; $CH_2$), 1,34-1,29 (m, 110H; $CH_2$), 0,51-0,40 (m, 4H; Si-$CH_2$), 0,02 (s, 250H; Si-$CH_3$).

**Beispiel 7**

*Herstellung von Polymerisations- und SL-Harzen*

Die in Tabellen 1 aufgeführten Komponenten wurden in den angegebenen Mengen homogen miteinander gemischt. Dazu wurden in einem Planetenmischer oder Speedmixer alle festen Komponenten (Blockcopolymer oder Kern-Schale-Partikel, Photoinitiator) in den Monomeren unter Rühren gelöst, gegebenenfalls auch unter Erwärmen auf 50 °C. Anschließend wurden die Urethandimethacrylattelechele zugegeben und bis zum Erreichen eines homogenen Gemisches weiter gerührt. Die Blockcopolymere ließen sich problemlos in die Mischungen einarbeiten. Zur Einarbeitung von Kern-Schale-Partikeln wurde zusätzlich mittels eines Rotor-Stator-Mischers (Ultra-Turrax T-25) bei einer Drehzahl von 3000 U/min. für 30 Minuten homogenisiert. Anschließend wurden die Mischungen im Planetenmischer entlüftet.

Mit den Formulierungen Nr. 5 und Nr. 6 wurden mit einem Stereolithographiedrucker (PrograPrint PR5, Fa. Ivoclar Vivadent AG, Schaan, Liechtenstein) im bottom-up-Prozess Prüfkörper hergestellt. Der Drucker belichtete die Proben in DLP-Technik mit einer Wellenlänge von 388 nm, einer Leistung von 10 mW/$cm^2$ und einer Pixelgröße von 50 $\mu$m im schichtweisen Aufbau. Die Schichtdicke betrug jeweils 100 $\mu$m. Anhaftendes Restharz wurde mit Isopropanol entfernt. Hierzu wurden die Prüfkörper zusammen mit der Bauplattform (PrograPrint Stage) mit einem PrograPrint Clean-Gerät (Fa. Ivoclar Vivadent AG, Schaan, Liechtenstein) in frischem Isopropanol unter Rühren zweimal gereinigt (erstes Bad 10 min, zweites Bad 5 min) und direkt im Anschluss mit Druckluft trockengeblasen. Danach wurden die Prüfkörper durch Belichten für 90 s mit Licht einer Wellenlänge von 405 nm nachvergütet. Dies erfolgte mittels PrograPrint Cure-Gerät (Fa. Ivoclar Vivadent AG, Schaan, Liechtenstein; Sofware: ProArt Print Splint, Jahr 2020). Anschließend wurden die Prüfkörper von der Bauplattform gelöst.

Mit den übrigen Zusammensetzungen aus Tabelle 1 wurden in Metallformen Prüfkörper präpariert, die mit einer dentalen Lichtquelle (PrograPrint Cure, Fa. Ivoclar Vivadent AG, Schaan, Liechtenstein; Sofware: ProArt Print Splint, Jahr: 2020) beidseitig bestrahlt und damit ausgehärtet wurden.

Die weitere Bearbeitung und Einlagerung der Prüfkörper erfolgte gemäss der einschlägigen Vorgaben der nachfolgend genannten Vorschriften. Die Biegefestigkeit (BF) und der Biege-E-Modul (BM) wurden nach der ISO-Norm ISO-4049 (Dentistry - Polymer-based filling, restorative and luting materials) bestimmt. Dazu wurden die Prüfkörper vorher entweder für 24 h bei Raumtemperatur trocken oder für 24 h bei 37 °C in Wasser gelagert. Zusätzlich wurden die Biegefestigkeit der Biegemodul nach der ISO-Norm 20795-1:2013 (Zahnheilkunde - Kunststoffe - Teil 1: Prothesenkunststoffe) gemessen. Danach wurden die Prüfkörper vor der Messung für 50 h bei 37 °C in deionisiertem Wasser gelagert und dann die Messung in einem thermostatisierten Becken unter Wasser bei 37 °C durchgeführt. Die Bestimmung der Bruchzähigkeit $K_{max}$ und der Brucharbeit FW erfolgten gemäß ISO 20795-1:2013. Die Ergebnisse der Messungen sind in Tabelle 2 wiedergegeben.

Die Formulierungen Nr. 1 und Nr. 10 sind Referenz-Beispiele, die weder Kern-Schale-Polymer noch Blockcopolymer enthalten. Diese Referenzbeispiele weisen zwar eine gute Biegefestigkeit und ein gutes Biegemodul auf, die Werte für die Bruchzähigkeit und die Brucharbeit sind jedoch schlecht und für die stereolithographische Anwendungszwecke unbrauchbar. Die Beispiele Nr. 3, Nr. 4 und Nr. 14 enthalten jeweils Kern-Schale-Polymer-Partikel. Die Zugabe der Partikel bewirkt in allen Fällen eine Verbessrung der Bruchzähigkeit ($K_{max}$ und Brucharbeit FW).

In Beispiel Nr. 14 wurde die Formulierung Nr. 10 mit 5 Gew.-% Kern-Schale-Partikel versetzt. Die Zugabe des Kern-Schale-Polymers bewirkt nicht nur eine Verbessrung der Bruchzähigkeit sondern auch eine deutliche Verschlechterung der Transparenz. In den Beispielen Nr. 12, Nr. 13 und Nr. 15 wurden jeweils 5 Gew.-% eines Blockcopolymers anstelle der Kern-Schale-Partikel als Schlagzähmodifikator zugefügt. Tabelle 2 zeigt, dass die Blockcopolymere in allen Fällen eine deutlich größere Verbesserung der Bruchzähigkeit als die Kern-Schale-Partikeln ergeben aber nur eine vergleichsweise geringe Verschlechterung der Transparenz zur Folge haben. Das Beispiel Nr. 11 zeigt, dass die Zugabe von 3 Gew.-% eines Blockcopolymers ausreichend ist, um eine ähnliche Verbesserung der Bruchzähigkeit wie 5 Gew.-% Kern-Schale-Partikeln zu erzielen.

Ein Vergleich der Beispiele Nr. 2 und Nr. 3 zeigt, dass auch hier die Zugabe von 3 Gew.-% Blockcopolymer in etwa die gleiche Verbesserung der Bruchzähigkeit wie 5 Gew.-% Kern-Schale-Partikel ergeben. Das Beispiel Nr. 2 zeichnet sich gegenüber Beispiel Nr. 3 durch eine höhere Transparenz aus und bestätigt, dass die Verwendung von Blockcopolymeren die Herstellung von Werkstoffen mit hoher Bruchzähigkeit und Transparenz ermöglicht.

Ein Vergleich der Beispiele Nr. 3 und Nr. 4 zeigt, dass durch eine Erhöhung der Menge an Kern-Schale-Partikeln von 5 auf 10 Gew.-% zwar eine weitere Verbesserung der Bruchzähigkeit möglich ist, diese aber mit einer zunehmenden Verschlechterung der Transparenz verbunden ist.

**Tabelle 1: Zusammensetzung der Polymerisations- und SL-Harze (Angaben in Gew.-%)**

| | Dimethacrylat | Urethandimeth-acrylat-Telechel | | Mono(meth)-acrylat | | | Photoinitiator | Kern-Schale-Partikel | Blockco-polymer | | |
|---|---|---|---|---|---|---|---|---|---|---|---|
| Batch | V380[a)] | Bsp. 2a | Bsp. 2b | Bsp. 1 | 2-PEMA[b)] | TCDA[c)] | TPO[d)] | MZ110[e)] | Bsp. 5 | Bsp. 6 | PCL-b-PDMS-b-PCL[f)] |
| Nr. 1*) | | 49,5 | | | 49,5 | | 1,0 | | | | |
| Nr. 2 | | 48,0 | | | 48,0 | | 1,0 | | | | 3,0 |
| Nr. 3*) | | 47,0 | | | 47,0 | | 1,0 | 5,0 | | | |
| Nr. 4*) | | 44,5 | | | 44,5 | | 1,0 | 10,0 | | | |
| Nr. 5 | | 47,0 | | | | 47,0 | 1,0 | | | | 5,0 |
| Nr. 6 | | 45,8 | | | | 45,8 | 1,0 | | | | 7,5 |
| Nr. 7 | | 48,0 | | 19,0 | 29,0 | | 1,0 | | | | 3,0 |
| Nr. 8 | | 47,0 | | 19,0 | 28,0 | | 1,0 | | | | 5,0 |
| Nr. 9 | 9,6 | 38,4 | | | 48,0 | | 1,0 | | | | 3,0 |
| Nr. 10*) | | | 49,5 | | 49,5 | | 1,0 | | | | |
| Nr. 11 | | | 48,0 | | 48,0 | | 1,0 | | | | 3,0 |
| Nr. 12 | | | 47,0 | | 47,0 | | 1,0 | | 5,0 | | |
| Nr. 13 | | | 47,0 | | 47,0 | | 1,0 | | | 5,0 | |
| Nr. 14*) | | | 47,0 | | 47,0 | | 1,0 | 5,0 | | | |
| Nr. 15 | | | 47,0 | | 47,0 | | 1,0 | | | | 5,0 |
| Nr. 16 | | | 33,0 | | 61,0 | | 1,0 | | | | 5,0 |

*) Vergleichsbeispiel

a) Additionsprodukt aus einer Mischung von 0,7 mol 2-Hydroxyethylmethacrylat und 0,3 mol 2-Hydroxypropylmethacrylat mit 1 mol α,α,α`,α`-Tetramethyl-m-xylylen-diisocyanat

b) 2-Phenoxyethylmethacrylat

c) Tricyclodecanmethylacrylat

d) Diphenyl(2,4,6-trimethylbenzoyl)phenylphosphinoxid (BASF)

e) Kern-Schale-Polymer-Partikel MZ110 (Kaneka)

f) PCL-b-PDMS-b-PCL Blockcopolymer, die Molmasse der PDMS-Blöcke ist 3200 g/mol und die Molmasse der PCL-Blöcke 1600 g/mol

**Tabelle 2: Eigenschaften der gehärteten Werkstoffe**

| | Viskosität1) | Biegefestigkeit bzw. -modul5) trocken ISO4049 | | Biegefestigkeit bzw. -modul6) in Wasser ISO4049 | | Biegefestigkeit bzw. -modul7) ISO20795-2 | | Bruchzähigkeit ISO2795-2 | | Glasübergangstemperatur3) $T_G$ | Netzwerkdichte4) $v_c$ | Transparenz2) |
|---|---|---|---|---|---|---|---|---|---|---|---|---|
| Batch | η [Pa·s] | BF [MPa] | BM [MPa] | BF [MPa] | BM [MPa] | BF [MPa] | BM [MPa] | $K_{max}$ [MPa·m$^{1/2}$] | FW [J/m$^2$] | [°C] | [mol/m$^3$] | [%] |
| Nr. 1*) | 1,2 | 128,6 | 3157 | 108,3 | 2642 | 86,7 | 2499 | 0,897 | 118 | 85,8 | 1365 | 88,6 |
| Nr. 2 | 1,5 | 89,4 | 2430 | 78,7 | 2208 | 71,4 | 2090 | 1,703 | 349 | 89,3 | 1660 | 81,0 |
| Nr. 3*) | 2,0 | 80,9 | 2307 | 86,5 | 2382 | 77,6 | 2077 | 1,732 | 370 | n.b. | n.b. | 76,1 |
| Nr. 4*) | n.b. | 106,5 | 2740 | 96,1 | 2451 | 56,2 | 1697 | 2,060 | 657 | n.b. | n.b. | 65,0 |
| Nr. 5 | n.b. | n.b. | n.b. | n.b. | n.b. | 59,1 | 1547 | 1,607 | 476 | n.b. | n.b. | n.b. |
| Nr. 6 | n.b. | n.b. | n.b. | n.b. | n.b. | 41,9 | 1036 | 1,604 | 616 | n.b. | n.b. | n.b. |
| Nr. 7 | 3,3 | 101,5 | 2729 | 87,9 | 2605 | 85,9 | 2553 | 1,610 | 287 | n.b. | n.b. | n.b. |
| Nr. 8 | 3,5 | 88,3 | 2424 | 86,9 | 2370 | 74,5 | 2148 | 2,082 | 577 | n.b. | n.b. | n.b. |
| Nr. 9 | 0,9 | 97,1 | 2753 | 88,0 | 2591 | 77,8 | 2273 | 1,481 | 255 | n.b. | n.b. | n.b. |
| Nr. 10*) | 4,2 | 117,2 | 3325 | 113,0 | 3103 | 85,2 | 3115 | 0,859 | 93 | 98,4 | 1060 | 90,9 |
| Nr. 11 | 4,4 | 100,5 | 3110 | 96,3 | 2950 | 88,5 | 2726 | 1,801 | 326 | n.b. | n.b. | 85,0 |
| Nr. 12 | 4,2 | 112,9 | 3221 | 104,7 | 2919 | 88,6 | 2507 | 2,068 | 489 | n.b. | n.b. | 88,2 |
| Nr. 13 | 4,8 | 109,1 | 3230 | 104,3 | 2855 | 88,5 | 2624 | 2,146 | 546 | n.b. | n.b. | 88,7 |
| Nr. 14*) | 3,7 | 105,9 | 3105 | 103,6 | 3170 | 65,1 | 2990 | 1,680 | 296 | n.b. | n.b. | 59,5 |
| Nr. 15 | 4,6 | 96,4 | 2858 | 96,3 | 2840 | 69,2 | 2192 | 2,010 | 613 | n.b. | n.b. | 83,0 |

(fortgesetzt)

| | Viskosität[1)] | Biegefestigkeit bzw. -modul[5)] trocken ISO4049 | | Biegefestigkeit bzw. -modul[6)] in Wasser ISO4049 | | Biegefestigkeit bzw. -modul[7)] ISO20795-2 | | Bruchzähigkeit ISO2795-2 | | Glasübergangstemperatur[3)] $T_G$ | Netzwerkdichte[4)] $v_c$ | Transparenz[2)] |
|---|---|---|---|---|---|---|---|---|---|---|---|---|
| Batch | $\eta$ [Pa·s] | BF [MPa] | BM [MPa] | BF [MPa] | BM [MPa] | BF [MPa] | BM [MPa] | $K_{max}$ [$MPa \cdot m^{1/2}$] | FW [$J/m^2$] | [°C] | [$mol/m^3$] | [%] |
| Nr. 16 | 4,1 | 107,1 | 3382 | 75,7 | 2381 | 62,8 | 1993 | 1,933 | 643 | n.b. | n.b. | 87,5 |

*) Vergleichsbeispiel

n.b. nicht bestimmt

1) Rotations-Viskosität, bestimmt mit einem Anton Paar Viskosimeter Modell MCR 302 mit CP25-2 Konus-Platte-Messmittel und einem Messspalt von 53 $\mu$m in Rotation bei einer Scherrate von 100/s bei 25°C

2) bestimmt mit einem Konika-Minolta Spektrophotometer Modell CM-5 an 1 mm dicken, auf Hochglanz polierten Prüfkörpern in Transmission (D65)

3) Speicher- und Verlustmodul eines Prüfkörpers (25 x 5 x 1 mm, längs eingespannt) wurden zwischen 25°C und 250°C mit einem Anton Paar Rheometer Modell MCR302 bestimmt (Frequenz 1 Hz, Deformation 0,05 %, Heizrate 2K / min); $T_G$ entspricht dem Maximum des Verlustfaktors tan $\delta$ (Verhältnis von Verlustmodul zu Speichermodul)

4) die Netzwerkdichte wurde nach der Formel $v_c = G'/(RT)$ berechnet, G' ist das Speichermodul bei der Temperatur $T_G$ + 50 K; R ist die allgemeine Gaskonstante; T ist die Temperatur bei $T_G$ + 50 K

5) Biegefestigkeit (BF) und Biegemodul (BM) gemessen gemäß ISO4049 nach trockener Lagerung für 24 h bei **RT**

6) Biegefestigkeit (BF) und Biegemodul (BM) gemessen gemäß ISO4049 nach Lagerung für 24 h bei 37°C in Wasser

7) Biegefestigkeit (BF) und Biegemodul (BM) gemessen gemäß ISO20795-2

## Patentansprüche

**1.** Radikalisch polymerisierbarer Dentalwerkstoff, der mindestens ein ABA- oder AB-Blockcopolymer (d), mindestens ein monofunktionelles, radikalisch polymerisierbares Monomer (a) und mindestens ein radikalisch polymerisierbares Urethandi(meth)acrylat-Telechel (b) enthält, **dadurch gekennzeichnet, dass** das Urethandi(meth)acrylat-Telechel (b) eine zahlenmittlere Molmasse von 750 bis 2000 g/mol hat und der allgemeinen Formel (II) entspricht,

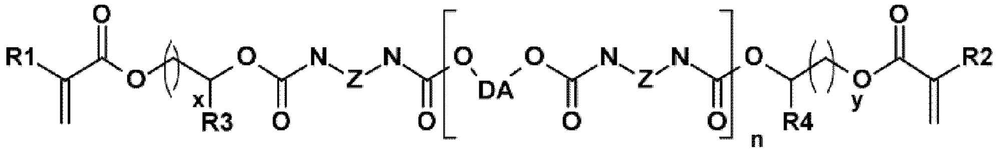

**Formel II**

in der die Variablen die folgenden Bedeutungen haben:

$R^1$, $R^2$ unabhängig voneinander jeweils H oder Methyl,
$R^3$, $R^4$ unabhängig voneinander jeweils H oder Methyl,
x, y unabhängig voneinander jeweils eine ganze Zahl von 1 bis 11,
n 1, 2 oder 3,
Z

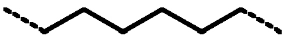
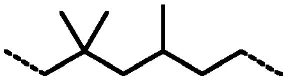
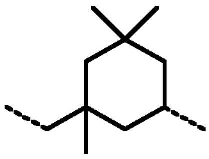
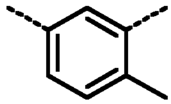
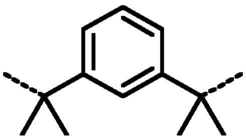
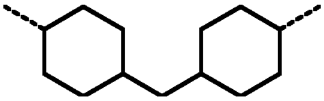
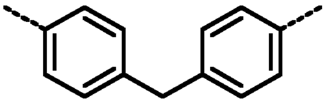

DA ein Strukturelement, das sich aus einem Diol HO-DA-OH durch Abspaltung der Wasserstoffatome der Hydroxylgruppen ableitet, wobei das Diol HO-DA-OH aus den folgenden Verbindungen ausgewählt ist: ethoxyliertes- oder propoxyliertes Bisphenol-A, o-Diphenyl oder p-Diphenyl mit 2 bis 6 Ethoxy- oder Propoxy-Gruppen, $C_2$-$C_{18}$-Alkandiole, die 1 bis 4 O- oder S-Atome in der Kohlenstoffkette enthalten können, cyclische oder polycyclische aliphatische Diole.

**2.** Radikalisch polymerisierbarer Dentalwerkstoff nach Anspruch 1, der

(a) 30 bis 70 Gew.-%, vorzugsweise 30 bis 61 Gew.-% und besonders bevorzugt 40 bis 60 Gew.-% mindestens eines aromatischen, bicyclischen oder tricyclischen Mono(meth)acrylats,
(b) 20 bis 60 Gew.-%, vorzugsweise 30 bis 55 Gew.-% und besonders bevorzugt 33 bis 55 Gew.-% mindestens eines Urethandi(meth)acrylat-Telechels mit einer zahlenmittleren Molmasse von 750 bis 2000 g/mol,
(c) 0 bis 30 Gew.-%, vorzugsweise 0 bis 20 Gew.% und besonders bevorzugt 0 Gew.-% Di(meth)acrylatmonomer(e),
(d) 1 bis 12 Gew.-%, vorzugsweise 2 bis 12 Gew.-% und besonders bevorzugt 2 bis 10 Gew.-% mindestens eines ABA- und/oder AB-Blockcopolymers, wobei der oder die A-Blöcke homogen mischbar und der B-Block nicht homogen mischbar mit der Mischung der Komponenten (a) bis (c) sind, und
(e) 0,1 bis 5,0 Gew.-%, vorzugsweise 0,2 bis 4,0 Gew.-% und besonders bevorzugt 0,3 bis 3,0 Gew.-% mindestens eines Initiators für die radikalische Polymerisation enthält,

jeweils bezogen auf die Gesamtmasse des Werkstoffs.

**3.** Dentalwerkstoff nach Anspruch 1, der als Monomer (a) mindestens ein aromatisches, bicyclisches oder tricyclisches Monomethacrylat der Formel (I) enthält,

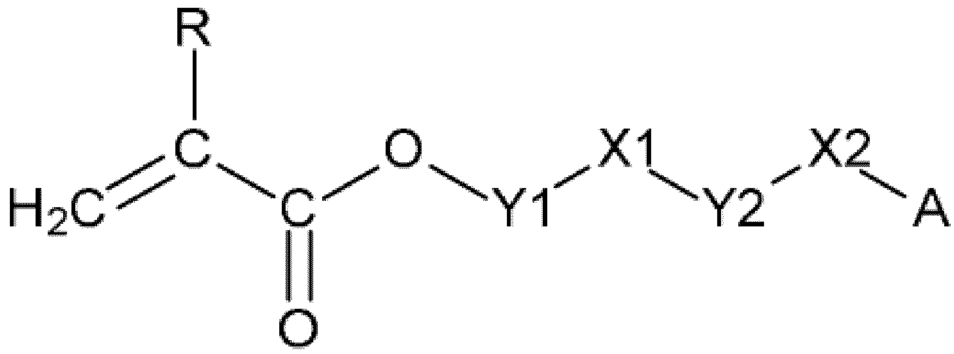

**Formel I**

in der die Variablen die folgenden Bedeutungen haben:

A eine aromatische Gruppe mit 6 bis 15 Kohlenstoffatomen oder eine bicyclische oder tricyclische aliphatische Gruppe mit 7 bis 10 Kohlenstoffatomen, wobei A unsubstituiert oder durch eine oder mehrere $C_1$-$C_5$-Alkyl-Gruppen, $C_1$-$C_5$-Alkoxy-Guppen und/oder Chloratome substituiert sein kann;
R Wasserstoff oder Methyl;
$X^1$, $X^2$ unabhängig voneinander jeweils entfällt oder eine Ether-, Ester- oder Urethangruppe, wobei $X^1$ entfällt wenn $Y^1$ entfällt und wobei $X^2$ entfällt wenn $Y^2$ entfällt;
$Y^1$, $Y^2$ unabhängig voneinander jeweils entfällt oder ein verzweigter oder vorzugsweise linearer aliphatischer Kohlenwasserstoffrest mit 1 bis 10 Kohlenstoffatomen, der durch 1 bis 3 Sauerstoffatome unterbrochen sein kann.

**4.** Dentalwerkstoff nach Anspruch 3, der als Komponente (a) 2-Phenoxyethyl-(meth)acrylat, 2-(o-Biphenyloxy) ethyl(meth)acrylat, 2-Hydroxy-3-phenoxy-propyl(meth)acrylat, Phenethyl(meth)acrylat, 2-[(Benzyloxycarbonyl)-amino]-ethyl(meth)acrylat, 2-[(Benzylcarbamoyl)-oxy]-ethyl(meth)acrylat, 1-Phenoxy-propan-2-yl-(meth)acrylat und 2-(p-Cumylphenoxy)-ethyl(meth)acrylat, 2-(Benzyloxy)ethyl(meth)acrylat, 3-Phenoxybenzyl(meth)acrylat, Phenoxypropyl-(meth)acrylat, 2-Benzyloxyethyl(meth)acrylat, 2-Benzoyloxyethyl(meth)acrylat, 2-(Meth)acryloyloxybenzoesäuremethylester, Tricylodecan(meth)acrylat, Tri-cyclodecanmethyl(meth)acrylat, 2-(p-Cumylphenoxy) ethyl(meth)acrylat, 4,7,7-Trimethylbicyclo[2.2.1]heptanyl(meth)acrylat, Octahydro-1H-4,7-methano-inden-5-yl-(meth)acrylat oder eine Mischung davon enthält.

**5.** Dentalwerkstoff nach einem der Ansprüche 1 bis 4, der als Komponente (b) ein Telechel gemäß der allgemeinen Formel (II) enthält, in der die Variablen die folgenden Bedeutungen haben:

$R^1$, $R^2$ jeweils Methyl,
$R^3$, $R^4$ jeweils Methyl ,
x, y unabhängig voneinander jeweils eine ganze Zahl von 1 bis 5,
n 1,
Z

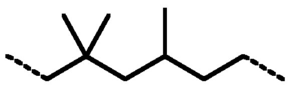 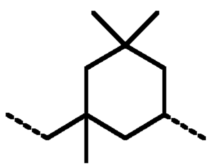 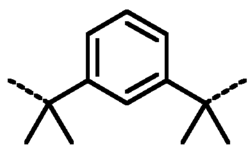

DA ein Strukturelement, das sich aus einem Diol HO-DA-OH durch Abspaltung der Wasserstoffatome der Hydroxylgruppen ableitet, wobei das Diol HO-DA-OH aus den folgenden Verbindungen ausgewählt ist: ethoxyliertes- oder propoxyliertes Bisphenol-A mit 2, 3 oder 4 Ethoxy- oder Propoxygruppen, Hexan-1,6-diol, Octan-1,8-diol, Nonan-1,9-diol, Decan-1,10-diol, Undecandiol oder Dodecan-1,12-diol, Tetra- oder Pentaethylenglycol, Cyclohexandiol, Norbornandiol, Tricyclodecandiol und Tricyclodecandimethanol (Octahydro-4,7-methano-1H-indendimethanol).

6. Dentalwerkstoff nach einem der Ansprüche 1 bis 5, der als **Komponente (d)** mindestens ein ABA- und/oder AB-Blockcopolymer enthält, bei dem der A-Block ein Oligomer ist, das aus einem oder mehreren der folgenden Monomere aufgebaut ist: cyclische, aliphatische Ester oder Ether, Arylenoxid, Alkylenoxid, radikalisch polymerisierbare Monomere, beispielsweise $\alpha,\beta$-ungesättigte Säuren und $\alpha,\beta$-ungesättigte Säureester; und der B-Block ein Polysiloxan- und/oder ein Polyvinyl- und/oder ein Polyalken- und/oder ein Polydien-Oligomer ist.

7. Dentalwerkstoff nach Anspruch 6, der als **Komponente (d)** mindestens ein ABA- und/oder AB-Blockcopolymer enthält, bei dem der A-Block ein oligomerer Polycaprolacton- (PCL), Poly(2,6-dimethyl-1,4-phenylenoxid)-, Poly(ethylenoxid)-, Poly(propylenoxid)- oder Poly(meth)acrylat-Baustein und der B-Block ein oligomerer Poly(dimethylsiloxan)- (PDMS), Poly(isopren)-, Poly-(vinylacetat)-, Poly(isobuten)-, cis-Poly(butadien)- oder Poly(ethylen)-Baustein ist.

8. Dentalwerkstoff nach Anspruch 7, der als **Komponente (d)** mindestens ein ABA-Triblockcopolymer des Typs PCL-b-PDMS-b-PCL und/oder PMMA-b-PDMS-b-PMMA mit einem Molverhältnis von A:B von 0,1 bis 5 und mit einer zahlenmittleren Molmasse von 3 bis 25 kDa, bevorzugt 4 bis 20 kDa und besonders bevorzugt 5 bis 10 kDa enthält.

9. Dentalwerkstoff nach einem der Ansprüche 1 bis 8, die als **Komponente (e)** mindestens einen Photoinitiator enthält, der vorzugsweise aus Benzophenon, Benzoin oder einem Derivat davon, einem $\alpha$-Diketon oder einem Derivat davon, wie 9,10-Phenanthrenchinon, 1-Phenyl-propan-1,2-dion, Diacetyl oder 4,4'-Dichlorbenzil, Campherchinon (CQ), 2,2-Dimethoxy-2-phenyl-acetophenon oder einem $\alpha$-Diketon in Kombination mit einem Amin als Reduktionsmittel, wie z.B. 4-(Dimethylamino)-benzoesäureester (EDMAB), N,N-Dimethylaminoethylmethacrylat, N,N-Dimethyl-sym.-xylidin oder Triethanolamin, Monoacyltrialkyl-, Diacyldialkyl-, Tetraacylgermanium, einem Tetraacylstannan, Benzoyltrimethylgermanium, Dibenzoyldiethylgermanium, Bis(4-methoxybenzoyl)diethylgermanium, Tetrakis(2-methylbenzoyl)germane oder Tetrakis-(mesitoyl)stannan oder einer Mischung davon,
einem Norrish-Typ-I-Photoinitiator, wie Acetophenone, z.B. 2,2-Diethoxy-1-phenylethanon, Benzoinether z.B. Irgacure 651 (Dimethylbenzilketal), Hydroxyalkylphenylacetophenone, z.B. Irgacure 184 (1-Hydroxy-cyclohexyl-phenylketon), einem Acyl- oder Bisacylphosphinoxid, z.B. Irgacure TPO (2,4,6-Trimethylbenzoyldiphenylphosphinoxid) oder Irgacure 819 (Bis(2,4,6-trimethylbenzoyl)phenylphosphinoxid), 2-Benzyl-2-(dimethylamino)-4'-morpholino-butyrophenon (Irgacure 369) und/oder 1-Butanon-2-(dimethylamino)-2-(4-methylphenyl)methyl-1-4-(4-morpholinyl) phenyl (Irgacure 379) ausgewählt ist.

10. Dentalwerkstoff nach einem der Ansprüche 1 bis 9, mit der folgenden Zusammensetzung:

(a) 30 bis 70 Gew.-%, bevorzugt 30 bis 61 Gew.-% besonders bevorzugt 40 bis 60 Gew.-% mindestens eines aromatischen, bicyclischen oder tricyclischen Mono(meth)acrylats,
(b) 20 bis 60 Gew.-%, bevorzugt 30 bis 55 Gew.-% und besonders bevorzugt 33 bis 55 Gew.-% mindestens eines Urethandi(meth)acrylat-Telechels mit einer zahlenmittleren Molmasse von 750 bis 2000 g/mol,
(c) 0 bis 30 Gew.-%, bevorzugt 0 bis 20 Gew.-%, und besonders bevorzugt 0 Gew.-% Di(meth)acrylatmonomer(e),
(d) 1 bis 12 Gew.-%, bevorzugt 2 bis 12 Gew.-% besonders bevorzugt 2 bis 10 Gew.-% mindestens eines ABA- bzw. AB-Blockcopolymers,
(e) 0,1 bis 5,0 Gew.-%, bevorzugt 0,2 bis 4,0 Gew.-% und besonders bevorzugt 0,3 bis 3,0 Gew.-% mindestens eines Initiators für die radikalische Polymerisation,
(f) 0 bis 15 Gew.-%, bevorzugt 0 bis 5 Gew.-% und besonders bevorzugt 0 Gew.-% Kern-Schale-Polymerpartikel,
(g) 0 bis 20 Gew.-%, vorzugsweise 0 bis 15 Gew.-% und besonders bevorzugt 0 bis 10 Gew.-% Füllstoff,
(h) 0 bis 1,0 Gew.-%, bevorzugt 0 bis 0,7 und besonders bevorzugt 0 bis 0,5 Gew.-% UV-Absorber,
(i) 0 bis 0,5 Gew.-%, bevorzugt 0 bis 0,1 Gew.-% und besonders bevorzugt 0 bis 0,05 Gew.-% optischen Aufheller und
(j) 0 bis 15 Gew.-%, bevorzugt 0 bis 10 Gew.-%, und besonders bevorzugt 0,2 bis 5 Gew.-% an weiteren Additiven,

jeweils bezogen auf die Gesamtmasse des Werkstoffs.

11. Dentalwerkstoff nach Anspruch 10, der

(a) 40 bis 61 Gew.-% an 2-Phenoxyethyl(meth)acrylat, 2-(o-Biphenyloxy)-ethyl(meth)acrylat, 2-Hydroxy-3-phenoxypropyl-(meth)acrylat, 2-[(Benzyloxycarbonyl)amino]ethyl(meth)acrylat, 1-Phenoxypropan-2yl-(meth)acrylat, 2-(Benzyloxy)-ethyl(meth)acrylat, 2-(Methaacryloyloxy)-ethyl(meth)acrylat, 3-Phenoxybenzyl(meth)acrylat, Phenoxypropyl-(meth)acrylat, 2-Benzyloxyethyl(meth)acrylat, 2-Benzoyloxyethyl(meth)-acrylat, 2-(Meth) acryloyloxybenzoesäuremethylester, 2-Phenylethyl-(meth)acrylat, Tricyclodecan(meth)acrylat, Tricyclodecanmethyl(meth)-acrylat und/oder 2-(p-Cumylphenoxy)ethylmethacrylat,
(b) 33 bis 55 Gew.-% mindestens eines Urethandi(meth)acrylat-Telechels mit einer zahlenmittleren Molmasse von 750-2000 g/mol und mit mindestens 4 Urethangruppen, hergestellt durch Reaktion von 1 Mol an ethoxyliertem oder propoxyliertem Bisphenol-A, Decan- oder Dodecandiol mit 2 Mol Isophorondiisocyanat (IPDI) und anschließender Umsetzung mit 2 Mol 2-Hydroxyethylmethacrylat (HEMA) oder Hydroxypropy-Imethacrylat (HPMA),
(c) 0 Gew.-% weitere Di(meth)acrylatmonomere,
(d) 2 bis 10 Gew.-% mindestens eines ABA- oder AB-Blockcopolymers, wobei der A-Block aus oligomeren Polycaprolacton-, Poly(2,6-dimethyl-1,4-phenylenoxid)-, Poly(ethylenoxid)-, Poly(propylenoxid)- oder Poly(meth)acrylat-Bausteinen und der B-Block aus Poly(di-methylsiloxan)-, Poly(isopren)-, Poly(vinylacetat)-, Poly(isobuten)-, cis-Poly(butadien)- oder Poly(ethylen)-Bausteinen aufgebaut ist,
(e) 0 % Kern-Schale-Polymere,
(f) 0,1 bis 5,0 Gew.-% mindestens eines Photoinitiators und
(g) 0,2 bis 5 Gew.-% eines oder mehrerer weitere Additiven enthält,

jeweils bezogen auf die Gesamtmasse des Werkstoffs.

**12.** Verfahren zur Herstellung von dentalen Formteilen bei dem man

(i) durch die direkte oder indirekte Digitalisierung des zu restaurierenden Zahns bzw. der zu restaurierenden Zähne am Computer ein virtuelles Abbild der Zahnsituation erstellt,
(ii) dann anhand dieses Abbilds am Computer ein Modell der Dentalrestauration oder Prothese konstruiert,
(iii) man dann eine Dentalwerkstoff gemäß einem der Ansprüche 1 bis 11 durch selektive Lichteinstrahlung schichtweise polymerisiert um die Dentalrestauration zu bilden und
(iv) man anschließend das Werkstücks ggf. durch Behandlung mit einem Lösungsmittel reinigt und
(v) man dann ggf. das Werkstücks durch Bestrahlen mit Licht und/oder durch Erwärmen auf eine Temperatur oberhalb von 50 °C weiter härtet.

**13.** Verwendung eines Werkstoffs gemäß einem der Ansprüche 1 bis 11 als Dentalwerkstoff oder zur Herstellung oder Reparatur von dentalen Formteilen.

**Claims**

**1.** Radically polymerizable dental material, which comprises at least one ABA or AB block copolymer (d), at least one monofunctional, radically polymerizable monomer (a) and at least one radically polymerizable urethane di(meth) acrylate telechel (b), **characterized in that** the urethane di(meth)acrylate telechel (b) has a number-average molar mass of from 750 to 2000 g/mol and corresponds to the general formula (II),

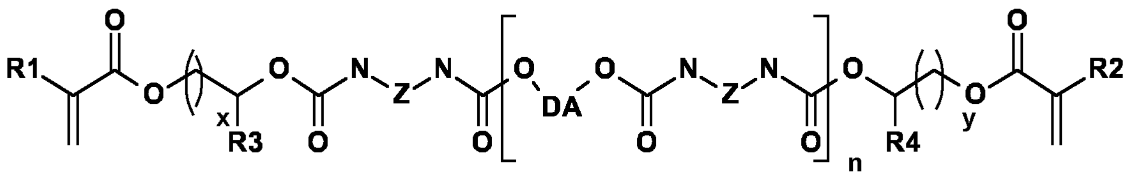

**Formula II**

in which the variables have the following meanings:

$R^1$, $R^2$ independently of each other in each case H or methyl,
$R^3$, $R^4$ independently of each other in each case H or methyl,
x, y independently of each other in each case an integer from 1 to 11,
n 1, 2 or 3,
Z

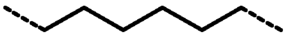
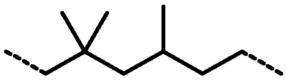
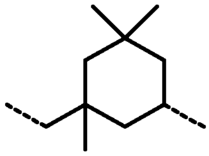
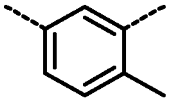
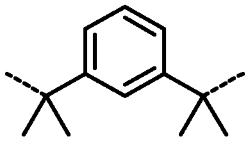
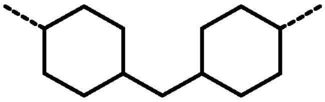
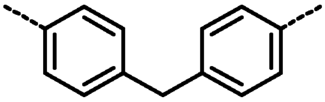

DA a structural element, which is derived from a diol HO-DA-OH by cleaving the hydrogen atoms from the hydroxyl groups, wherein the diol HO-DA-OH is selected from the following compounds: ethoxylated or propoxylated bisphenol A, o-diphenyl or p-diphenyl with 2 to 6 ethoxy or propoxy groups, $C_2$-$C_{18}$ alkanediols, which can contain 1 to 4 O or S atoms in the carbon chain, cyclic or polycyclic aliphatic diols.

**2.** Radically polymerizable dental material according to claim 1, which comprises

(a) 30 to 70 wt.-%, preferably 30 to 61 wt.-% and particularly preferably 40 to 60 wt.-% of at least one aromatic, bicyclic or tricyclic mono(meth)acrylate,
(b) 20 to 60 wt.-%, preferably 30 to 55 wt.-% and particularly preferably 33 to 55 wt.-% of at least one urethane di(meth)acrylate telechel with a number-average molar mass of from 750 to 2000 g/mol,
(c) 0 to 30 wt.-%, preferably 0 to 20 wt.-% and particularly preferably 0 wt.-% of di(meth)acrylate monomer(s),
(d) 1 to 12 wt.-%, preferably 2 to 12 wt.-% and particularly preferably 2 to 10 wt.-% of at least one ABA and/or AB block copolymer, wherein the A block or blocks are homogeneously miscible with the mixture of components (a) to (c) and the B block is not homogeneously miscible with the mixture of components (a) to (c), and
(e) 0.1 to 5.0 wt.-%, preferably 0.2 to 4.0 wt.-% and particularly preferably 0.3 to 3.0 wt.-% of at least one initiator for the radical polymerization,

in each case relative to the total mass of the material.

**3.** Dental material according to claim 1, which comprises as monomer (a) at least one aromatic, bicyclic or tricyclic monomethacrylate of Formula (I)

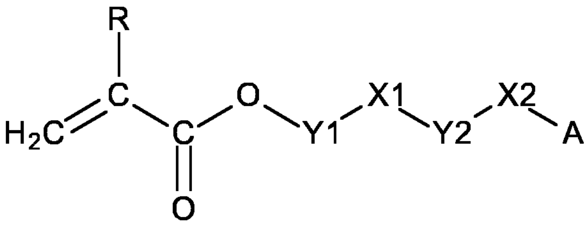

**Formula I**

in which the variables have the following meanings:

A an aromatic group with 6 to 15 carbon atoms or a bicyclic or tricyclic aliphatic group with 7 to 10 carbon atoms, wherein A can be unsubstituted or substituted by one or more $C_1$-$C_5$ alkyl groups, $C_1$-$C_5$ alkoxy groups and/or chlorine atoms;
R hydrogen or methyl;
$X^1$, $X^2$ are each independently absent or an ether, ester or urethane group, wherein $X^1$ is absent if $Y^1$ is absent and wherein $X^2$ is absent if $Y^2$ is absent;
$Y^1$, $Y^2$ are each independently absent or a branched or preferably linear aliphatic hydrocarbon radical with 1 to 10 carbon atoms, which can be interrupted by 1 to 3 oxygen atoms.

**4.** Dental material according to claim 3, which comprises as component (a) 2-phenoxyethyl (meth)acrylate, 2-(o-biphenyloxy)ethyl (meth)acrylate, 2-hydroxy-3-phenoxypropyl (meth)acrylate, phenethyl (meth)acrylate, 2-[(benzyloxycarbonyl)-amino]-ethyl (meth)acrylate, 2-[(benzylcarbamoyl)-oxy]-ethyl (meth)acrylate, 1-phenoxypropan-2-yl (meth)acrylate and 2-(p-cumylphenoxy)-ethyl (meth)acrylate, 2-(benzyloxy)ethyl (meth)acrylate, 3-phenoxybenzyl

(meth)acrylate, phenoxypropyl (meth)acrylate, 2-benzyloxyethyl (meth)acrylate, 2-benzoyloxyethyl (meth)acrylate, 2-(meth)acryloyloxybenzoic acid methyl ester, tricyclodecane (meth)acrylate, tricyclodecane methyl (meth)acrylate, 2-(p-cumylphenoxy)ethyl (meth)acrylate, 4,7,7-trimethylbicyclo[2.2.1]heptanyl (meth)acrylate, octahydro-1H-4,7-methanoinden-5-yl (meth)acrylate or a mixture thereof.

**5.** Dental material according to any one of claims 1 to 4, which comprises as component (b) a telechel according to the general formula (II), in which the variables have the following meanings:

$R^1$, $R^2$ each methyl,
$R^3$, $R^4$ each methyl ,
x, y independently of each other, each an integer from 1 to 5,
n 1,
Z

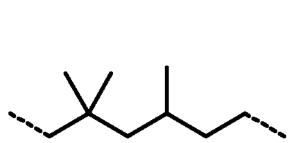
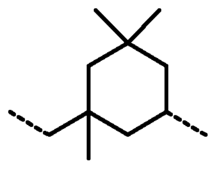
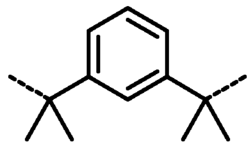

DA a structural element, which is derived from a diol HO-DA-OH by cleaving the hydrogen atoms from the hydroxyl groups, wherein the diol HO-DA-OH is selected from the following compounds: ethoxylated or propoxylated bisphenol A with 2, 3 or 4 ethoxy or propoxy groups, hexane-1,6-diol, octane-1,8-diol, nonane-1,9-diol, decane-1,10-diol, undecanediol or dodecane-1,12-diol, tetra- or pentaethylene glycol, cyclohexanediol, norbornanediol, tri-cyclodecanediol and tricyclodecanedimethanol (octahydro-4,7-methano-1H-indenedimethanol).

**6.** Dental material according to one of claims 1 to 5, which comprises as **component (d)** at least one ABA and/or AB block copolymer, in which the A block is an oligomer which is made up of one or more of the following monomers: cyclic, aliphatic esters or ethers, arylene oxide, alkylene oxide, radically polymerizable monomers, for example $\alpha,\beta$-unsaturated acids and $\alpha,\beta$-unsaturated acid esters; and the B block is a polysiloxane oligomer and/or a polyvinyl oligomer and/or a polyalkene oligomer and/or a polydiene oligomer.

**7.** Dental material according to claim 6, which comprises as **component (d)** at least one ABA and/or AB block copolymer, in which the A block is an oligomeric polycaprolactone (PCL), poly(2,6-dimethyl-1,4-phenylene oxide), poly(ethylene oxide), poly(propylene oxide) or poly(meth)acrylate building block and the B block is an oligomeric poly(dimethylsiloxane) (PDMS), poly(isoprene), poly(vinyl acetate), poly(isobutene), cis-poly(butadiene) or poly(ethylene) building block.

**8.** Dental material according to claim 7, which comprises as **component (d)** at least one ABA triblock copolymer of the PCL-b-PDMS-b-PCL and/or PMMA-b-PDMS-b-PMMA type with a molar ratio of A:B of from 0.1 to 5 and with a molar mass of from 3 to 25 kDa, preferably 4 to 20 kDa and particularly preferably 5 to 10 kDa.

**9.** Dental material according to one of claims 1 to 8, which comprises as **component (e)** at least one photoinitiator, which is preferably selected from benzophenone, benzoin or a derivative thereof, an $\alpha$-diketone or a derivative thereof, such as 9,10-phenanthrenequinone, 1-phenyl-propane-1,2-dione, diacetyl or 4,4'-dichlorobenzil, camphorquinone (CQ), 2,2-dimethoxy-2-phenylacetophenone or an $\alpha$-diketone in combination with an amine as reducing agent, such as e.g. 4-(dimethylamino)-benzoic acid ester (EDMAB), N,N-dimethylaminoethyl methacrylate, N,N-dimethyl-sym.-xylidine or triethanolamine, monoacyltrialkyl-, diacyldialkyl-, tetraacylgermanium, a tetraacylstannane, benzoyltrimethylgermanium, dibenzoyldiethylgermanium, bis(4-methoxybenzoyl)diethylgermanium, tetrakis(2-methylbenzoyl)germane or tetrakis(mesitoyl)stannane or a mixture thereof,
a Norrish type I photoinitiator, such as acetophenones, e.g. 2,2-diethoxy-1-phenylethanone, benzoin ethers e.g. Irgacure 651 (benzil dimethyl ketal), hydroxyalkylphenylacetophenones, e.g. Irgacure 184 (1-hydroxycyclohexyl phenyl ketone), an acyl- or bisacylphosphine oxide, e.g. Irgacure TPO (2,4,6-trimethylbenzoyldiphenylphosphine oxide) or Irgacure 819 (bis(2,4,6-trimethylbenzoyl)phenylphosphine oxide), 2-benzyl-2-(dimethylamino)-4'-morpholinobutyrophenone (Irgacure 369) and/or 1-butanone-2-(dimethylamino)-2-(4-methylphenyl)methyl-1-4-(4-morpholinyl)phenyl (Irgacure 379).

**10.** Dental material according to one of claims 1 to 9, with the following composition:

(a) 30 to 70 wt.-%, preferably 30 to 61 wt.-%, particularly preferably 40 to 60 wt.-% of at least one aromatic, bicyclic or tricyclic mono(meth)acrylate,
(b) 20 to 60 wt.-%, preferably 30 to 55 wt.-% and particularly preferably 33 to 55 wt.-% of at least one urethane di(meth)acrylate telechel with a number-average molar mass of from 750 to 2000 g/mol,
(c) 0 to 30 wt.-%, preferably 0 to 20 wt.-% and particularly preferably 0 wt.-% of di(meth)acrylate monomer(s),
(d) 1 to 12 wt.-%, preferably 2 to 12 wt.-%, particularly preferably 2 to 10 wt.-% of at least one ABA or AB block copolymer,
(e) 0.1 to 5.0 wt.-%, preferably 0.2 to 4.0 wt.-% and particularly preferably 0.3 to 3.0 wt.-% of at least one initiator for the radical polymerization,
(f) 0 to 15 wt.-%, preferably 0 to 5 wt.-% and particularly preferably 0 wt.-% of core-shell polymer particles,
(g) 0 to 20 wt.-%, preferably 0 to 15 wt.-% and particularly preferably 0 to 10 wt.-% of filler,
(h) 0 to 1.0 wt.-%, preferably 0 to 0.7 and particularly preferably 0 to 0.5 wt.-% of UV absorber,
(i) 0 to 0.5 wt.-%, preferably 0 to 0.1 wt.-% and particularly preferably 0 to 0.05 wt.-% of optical brightener and
(j) 0 to 15 wt.-%, preferably 0 to 10 wt.-% and particularly preferably 0.2 to 5 wt.-% of further additives,

in each case relative to the total mass of the material.

**11.** Dental material according to claim 10, which comprises

(a) 40 to 61 wt.-% of 2-phenoxyethyl (meth)acrylate, 2-(o-biphenyloxy)ethyl (meth)acrylate, 2-hydroxy-3-phenoxypropyl (meth)acrylate, 2-[(benzyloxycarbonyl)amino]ethyl (meth)acrylate, 1-phenoxypropan-2-yl (meth) acrylate, 2-(benzyloxy)-ethyl (meth)acrylate, 2-(methacryloyloxy)ethyl (meth)acrylate, 3-phenoxybenzyl (meth)acrylate, phenoxypropyl (meth)acrylate, 2-benzyloxyethyl (meth)acrylate, 2-benzoyloxyethyl (meth)acrylate, 2-(meth)acryloyloxybenzoic acid methyl ester, 2-phenylethyl (meth)acrylate, tricyclodecane (meth)acrylate, tricyclodecane methyl (meth)acrylate and/or 2-(p-cumylphenoxy)ethyl methacrylate,
(b) 33 to 55 wt.-% of at least one urethane di(meth)acrylate telechel with a number-average molar mass of 750-2000 g/mol and with at least 4 urethane groups, prepared by reacting 1 mol ethoxylated or propoxylated bisphenol A, decanediol or dodecanediol with 2 mol isophorone diisocyanate (IPDI) and then reacting with 2 mol 2-hydroxyethyl methacrylate (HEMA) or hydroxypropyl methacrylate (HPMA),
(c) 0 wt.-% further di(meth)acrylate monomers,
(d) 2 to 10 wt.-% of at least one ABA or AB block copolymer, wherein the A block is made up of oligomeric polycaprolactone, poly(2,6-dimethyl-1,4-phenylene oxide), poly(ethylene oxide), poly(propylene oxide) or poly(meth)acrylate building blocks and the B block is made up of poly(dimethylsiloxane), poly(isoprene), poly(vinyl acetate), poly(isobutene), cis-poly(butadiene) or poly(ethylene) building blocks,
(e) 0% of core-shell polymers,
(f) 0.1 to 5.0 wt.-% of at least one photoinitiator and
(g) 0.2 to 5 wt.-% of one or more further additives,

in each case relative to the total mass of the material.

**12.** Process for the production of dental shaped parts in which

(i) a virtual image of the tooth situation is created by direct or indirect digitization of the tooth to be restored or of the teeth to be restored on a computer,
(ii) then a model of the dental restoration or prosthesis is constructed on the computer on the basis of this image,
(iii) a dental material in accordance with one of claims 1 to 11 is then polymerized in layers by selective light irradiation in order to form the dental restoration and
(iv) the workpiece is then cleaned, optionally by treatment with a solvent, and
(v) the workpiece is then optionally further cured by irradiating with light and/or by heating to a temperature above 50°C.

**13.** Use of a material in accordance with one of claims 1 to 11 as dental material or for the production or repair of dental shaped parts.

## Revendications

**1.** Matériau de dentisterie polymérisable par voie radicalaire, qui contient au moins un copolymère à blocs ABA ou AB (d), au moins un monomère monofonctionnel polymérisable par voie radicalaire (a) et au moins un composé téléchélique di(méth)acrylate d'uréthane polymérisable par voie radicalaire (b), **caractérisé en ce que** le composé téléchélique di(méth)acrylate d'uréthane (b) présente une masse molaire moyenne en nombre de 750 à 2000 g/mol et correspond à la formule générale (II)

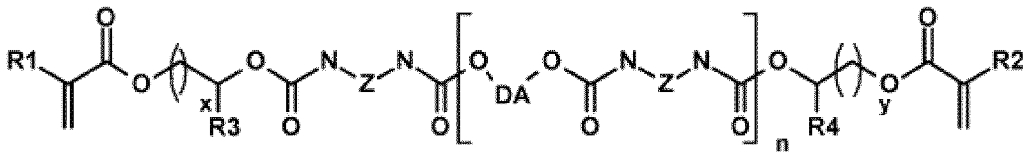

Formule II

dans laquelle les symboles ont les significations suivantes :

- $R^1$ et $R^2$ représentent chacun, indépendamment l'un de l'autre, un atome d'hydrogène ou un groupe méthyle,
- $R^3$ et $R^4$ représentent chacun, indépendamment l'un de l'autre, un atome d'hydrogène ou un groupe méthyle,
- x et y représentent chacun, indépendamment l'un de l'autre, un nombre entier valant de 1 à 11,
- l'indice n vaut 1, 2 ou 3,
- Z représente

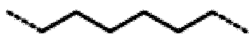 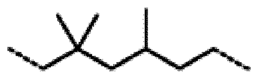 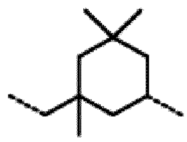 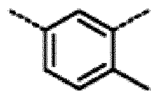
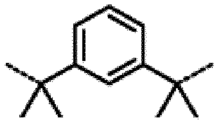 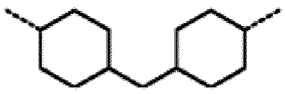 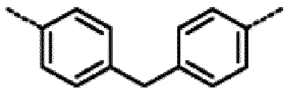

- et DA représente un fragment structural qui dérive d'un diol HO-DA-OH par enlèvement des atomes d'hydrogène des groupes hydroxyle, étant entendu que le diol HO-DA-OH est choisi parmi les composés suivants : bisphénol A, ortho-diphényle ou paradiphényle, éthoxylé ou propoxylé, comportant de 2 à 6 groupes éthoxy ou propoxy, alcanediols en $C_2$-$C_{18}$, dont la chaîne d'atomes de carbone peut comprendre de 1 à 4 atomes d'oxygène ou de soufre, et diols aliphatiques cycliques ou polycycliques.

**2.** Matériau de dentisterie polymérisable par voie radicalaire, conforme à la revendication 1, qui contient

a) de 30 à 70 % en poids, de préférence de 30 à 61 % en poids, et mieux encore de 40 à 60 % en poids, d'au moins un mono(méth)-acrylate aromatique, bicyclique ou tricyclique,
b) de 20 à 60 % en poids, de préférence de 30 à 55 % en poids, et mieux encore de 33 à 55 % en poids, d'au moins un composé téléchélique di(méth)acrylate d'uréthane présentant une masse molaire moyenne en nombre de 750 à 2000 g/mol,
c) de 0 à 30 % en poids, de préférence de 0 à 20 % en poids, et mieux encore 0 % en poids, de monomère(s) di(méth)acrylate(s),
d) de 1 à 12 % en poids, de préférence de 2 à 12 % en poids, et mieux encore de 2 à 10 % en poids, d'au moins un copolymère à blocs ABA et/ou AB, étant entendu que le ou les bloc(s) A est ou sont miscible(s) jusqu'à homogénéité au mélange des composants (a) à (c) et que le bloc B ne l'est pas,
e) et de 0,1 à 5,0 % en poids, de préférence de 0,2 à 4,0 % en poids, et mieux encore de 0,3 à 3,0 % en poids, d'au moins un amorceur de polymérisation par voie radicalaire,

dans chaque cas par rapport à la masse totale du matériau.

**3.** Matériau de dentisterie conforme à la revendication 1, qui contient, en tant que monomère (a), au moins un monométhacrylate aromatique, bicyclique ou tricyclique de formule I :

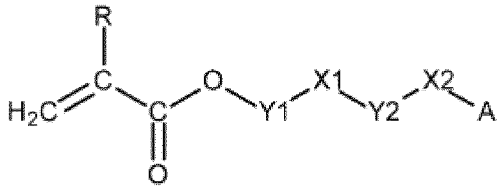

Formule I

dans laquelle les symboles ont les significations suivantes :

- A représente un groupe aromatique comportant de 6 à 15 atomes de carbone ou un groupe aliphatique bicyclique ou tricyclique comportant de 7 à 10 atomes de carbone, étant entendu que ce groupe A peut ne porter aucun substituant ou porter en tant que substituant(s) un ou plusieurs groupe(s) alkyle en $C_1$-$C_5$, groupe(s) alcoxy en $C_1$-$C_5$ et/ou atome(s) de chlore,
- R représente un atome d'hydrogène ou un groupe méthyle,
- $X^1$ et $X^2$, indépendamment l'un de l'autre, ne représentent rien ou représentent chacun un groupe ester, éther ou uréthane, étant entendu que $X^1$ ne représente rien si $Y^1$ ne représente rien et que $X^2$ ne représente rien si $Y^2$ ne représente rien,
- et $Y^1$ et $Y^2$, indépendamment l'un de l'autre, ne représentent rien ou représentent chacun un reste d'hydrocarbure aliphatique, ramifié ou de préférence linéaire, qui comporte de 1 à 10 atomes de carbone et dont la chaîne peut être interrompue par 1 à 3 atome(s) d'oxygène.

**4.** Matériau de dentisterie conforme à la revendication 3, qui contient, en tant que composant (a), du (méth)acrylate de 2-phénoxy-éthyle, du (méth)acrylate de 2-(o-biphényl-oxy)-éthyle, du (méth)acrylate de 2-hydroxy-3-phénoxy-propyle, du (méth)acrylate de phénéthyle, du (méth)acrylate de 2-[(benzyl-oxy-carbonyl)-amino)]-éthyle, du (méth)acrylate de 2-[(benzyl-carbamyl)-oxy)]-éthyle, du (méth)acrylate de 1-phénoxy-prop-2-yle, du (méth)acrylate de 2-(p-cumyl-phénoxy)-éthyle, du (méth)acrylate de 2-(benzyl-oxy)-éthyle, du (méth)-acrylate de 3-phénoxy-benzyle, du (méth) acrylate de phénoxy-propyle, du (méth)acrylate de 2-(benzyl-oxy)-éthyle, du (méth)acrylate de 2-(benzoyl-oxy)-éthyle, de l'ester méthylique d'acide 2-(méth)acryloyloxy-benzoïque, du (méth)acrylate de tricyclodécyle, du (méth) acrylate de tricyclodécyl-méthyle, du (méth)acrylate de 2-(p-cumyl-phénoxy)-éthyle, du (méth)acrylate de 4,7,7-triméthyl-bicyclo[2.2.1]heptyle, du (méth)acrylate d'octahydro-1H-4,7-méthano-indén-5-yle, ou un mélange de ces composés.

**5.** Matériau de dentisterie conforme à l'une des revendications 1 à 4, qui contient, en tant que composant (b), un composé téléchélique de formule générale (II) dans laquelle les symboles ont les significations suivantes :

- $R^1$ et $R^2$ représentent chacun un groupe méthyle,
- $R^3$ et $R^4$ représentent chacun un groupe méthyle,
- x et y représentent chacun, indépendamment l'un de l'autre, un nombre entier valant de 1 à 5,
- l'indice n vaut 1,
- Z représente

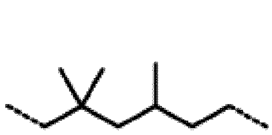 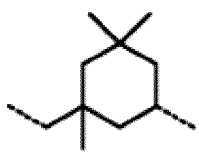 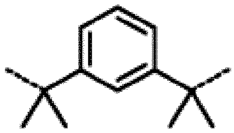

- et DA représente un fragment structural qui dérive d'un diol HO-DA-OH par enlèvement des atomes d'hydrogène des groupes hydroxyle, étant entendu que le diol HO-DA-OH est choisi parmi les composés suivants : bisphénol A éthoxylé ou propoxylé comportant 2, 3 ou 4 groupes éthoxy ou propoxy, hexane-1,6-diol, octane-1,8-diol, nonane-1,9-diol, décane-1,10-diol, undécanediol, dodécane-1,12-diol, tétraéthylèneglycol, pentaéthylèneglycol, cyclohexanediol, norbornanediol, tricyclodécanediol et tricyclodécanediméthanol (octahydro-4,7-méthano-1H-indène-diméthanol).

**6.** Matériau de dentisterie conforme à l'une des revendications 1 à 5, qui contient, en tant que composant (d), au moins

un copolymère à blocs ABA et/ou AB dans lequel le bloc A est un oligomère formé à partir de l'un ou plusieurs des monomères suivants : esters ou éthers aliphatiques cycliques, oxydes d'arylène, oxydes d'alkylène, monomères polymérisables par voie radicalaire, par exemple acides $\alpha,\beta$-insaturés et esters d'acide $\alpha,\beta$-insaturé, et le bloc B est un oligomère polysiloxane et/ou un oligomère polyvinylique et/ou un oligomère polyalcène et/ou un oligomère polydiène.

**7.** Matériau de dentisterie conforme à la revendication 6, qui contient, en tant que composant (d), au moins un copolymère à blocs ABA et/ou AB dans lequel le bloc A est un bloc constituant oligomère de type polycaprolactone (PCL), poly(oxy-2,6-diméthyl-1,4-phénylène), poly(oxy-éthylène), poly(oxy-propylène) ou poly(méth)acrylate, et le bloc B est un bloc constituant oligomère de type poly(diméthyl-siloxane) (PDMS), polyisoprène, poly(acétate de vinyle), polyisobutène, cis-polybutadiène, ou polyéthylène.

**8.** Matériau de dentisterie conforme à la revendication 7, qui contient, en tant que composant (d), au moins un copolymère tribloc ABA de type PCL-b-PDMS-b-PCL et/ou PMMA-b-PDMS-b-PMMA présentant un rapport molaire A/B valant de 0,1 à 5 et une masse molaire moyenne en nombre valant de 3 à 25 kDa, de préférence de 4 à 20 kDa et mieux encore de 5 à 10 kDa.

**9.** Matériau de dentisterie conforme à l'une des revendications 1 à 8, qui contient, en tant que composant (e), au moins un photoamorceur choisi de préférence parmi les suivants : benzophénone, benzoïne ou l'un de ses dérivés, une $\alpha$-dicétone ou un dérivé d'$\alpha$-dicétone, telles les 9,10-phénanthrène-quinone, 1-phényl-propane-1-2-dione, diacétyle, 4,4'-dichloro-benzile, camphoquinone (CQ) et 2,2-diméthoxy-2-phényl-acétophénone, ou une $\alpha$-dicétone en combinaison avec une amine servant d'agent réducteur, comme par exemple les 4-(diméthyl-amino)-benzoate (EDMAB), méthacrylate de N,N-diméthyl-amino-éthyle, N,N-diméthyl-sym-xylidine et triéthanolamine, les monoacyl-trialkyl-germanium, di-acyl-dialkyl-germanium et tétraacyl-germanium, un tétraacyl-stannane, benzoyl-triméthyl-germanium, dibenzoyl-diéthyl-germanium, bis(4-méthoxy-benzoyl)-diéthyl-germanium, tétrakis(2-méthyl-benzoyl)-germane ou tétrakis(mésitoyl)-stannane, ou un mélange de ceux-ci, un photoamorceur de Norrish de type I, comme les acétophénones, par exemple la 2,2-diéthoxy-1-phényl-éthanone, les éthers de benzoïne, par exemple Irgacure 651 (diméthyl-cétal de benzile), les hydroxyalkyl-phényl-acétophénones, par exemple Irgacure 184 ((1-hydroxy-cyclohexyl)-phényl-cétone), un oxyde d'acyl-phosphine ou de bis-acyl-phosphine, par exemple Irgacure TPO (oxyde de (2,4,6-triméthyl-benzoyl)-diphényl-phosphine) ou Irgacure 819 (oxyde de bis(2,4,6-triméthyl-benzoyl)-phényl-phosphine), la 2-benzyl-2-(diméthyl-amino)-4'-morpholino-butyrophénone (Irgacure 369) et/ou la 2-(diméthyl-amino)-2-[(4-méthyl-phényl)-méthyl]-1-[4-(morpholin-4-yl)-phényl]-butane-1-one (Irgacure 379).

**10.** Matériau de dentisterie conforme à l'une des revendications 1 à 9, présentant la composition suivante :

a) de 30 à 70 % en poids, de préférence de 30 à 61 % en poids, et mieux encore de 40 à 60 % en poids, d'au moins un mono(méth)-acrylate aromatique, bicyclique ou tricyclique,
b) de 20 à 60 % en poids, de préférence de 30 à 55 % en poids, et mieux encore de 33 à 55 % en poids, d'au moins un composé téléchélique di(méth)acrylate d'uréthane présentant une masse molaire moyenne en nombre de 750 à 2000 g/mol,
c) de 0 à 30 % en poids, de préférence de 0 à 20 % en poids, et mieux encore 0 % en poids, de monomère(s) di(méth)acrylate(s),
d) de 1 à 12 % en poids, de préférence de 2 à 12 % en poids, et mieux encore de 2 à 10 % en poids, d'au moins un copolymère à blocs ABA ou AB,
e) de 0,1 à 5,0 % en poids, de préférence de 0,2 à 4,0 % en poids, et mieux encore de 0,3 à 3,0 % en poids, d'au moins un amorceur de polymérisation par voie radicalaire,
f) de 0 à 15 % en poids, de préférence de 0 à 5 % en poids, et mieux encore 0 % en poids, de particules en polymère de type noyau-coque,
g) de 0 à 20 % en poids, de préférence de 0 à 15 % en poids, et mieux encore de 0 à 10 % en poids, d'une charge,
h) de 0 à 1,0 % en poids, de préférence de 0 à 0,7 % en poids, et mieux encore de 0 à 0,5 % en poids, d'un absorbeur d'UV,
i) de 0 à 0,5 % en poids, de préférence de 0 à 0,1 % en poids, et mieux encore de 0 à 0,05 % en poids, d'un azurant optique,
j) et de 0 à 15 % en poids, de préférence de 0 à 10 % en poids, et mieux encore de 0,2 à 5 % en poids, d'autres adjuvants,

dans chaque cas par rapport à la masse totale du matériau.

**11.** Matériau de dentisterie conforme à la revendication 10, qui contient :

a) de 40 à 61 % en poids de (méth)acrylate de 2-phénoxy-éthyle, de (méth)acrylate de 2-(o-biphényl-oxy)-éthyle, de (méth)acrylate de 2-hydroxy-3-phénoxy-propyle, de (méth)acrylate de 2-[(benzyl-oxy-carbonyl)-amino)]-éthyle, de (méth)acrylate de 1-phénoxy-prop-2-yle, de (méth)acrylate de 2-(benzyl-oxy)-éthyle, de (méth)-acrylate de méthacryloyl-oxy-éthyle, de (méth)acrylate de 3-phénoxy-benzyle, de (méth)acrylate de phénoxy-propyle, de (méth)-acrylate de 2-(benzyl-oxy)-éthyle, de (méth)acrylate de 2-(benzoyl-oxy)-éthyle, d'ester méthylique d'acide 2-(méth)acryloyl-oxy-benzoïque, de (méth)acrylate de 2-phényl-éthyle, de (méth)acrylate de tricyclodécyle, de (méth)acrylate de tricyclodécyl-méthyle, et/ou de méthacrylate de 2-(p-cumyl-phénoxy)-éthyle,
b) de 33 à 55 % en poids, d'au moins un composé téléchélique di(méth)acrylate d'uréthane présentant une masse molaire moyenne en nombre de 750 à 2000 g/mol et comportant au moins 4 groupes uréthane, préparé par réaction de 1 mole de bisphénol A éthoxylé ou propoxylé, de décanediol ou de dodécanediol avec 2 moles d'isophorone-diisocyanate (IPDI) et conversion ultérieure à l'aide de 2 moles de méthacrylate de 2-hydroxy-éthyle (HEMA) ou de méthacrylate d'hydroxy-propyle (HPMA),
c) 0 % en poids d'autres monomères di(méth)acrylates,
d) de 2 à 10 % en poids, d'au moins un copolymère à blocs ABA ou AB, dans lequel le bloc A est un bloc constituant oligomère de type polycaprolactone, poly(oxy-2,6-diméthyl-1,4-phénylène), poly(oxy-éthylène), poly(oxy-propylène) ou poly(méth)acrylate, et le bloc B est un bloc constituant de type poly(diméthyl-siloxane), polyisoprène, poly(acétate de vinyle), polyisobutène, cis-polybutadiène, ou polyéthylène,
e) 0 % de polymère de type noyau-coque,
f) de 0,1 à 5,0 % en poids d'au moins un photoamorceur,
g) et de 0,2 à 5 % en poids d'un ou plusieurs autre(s) adjuvant(s), dans chaque cas par rapport à la masse totale du matériau.

**12.** Procédé de fabrication de pièces façonnées de dentisterie , dans lequel

i) on produit sur ordinateur, par numérisation directe ou indirecte de la dent à restaurer ou des dents à restaurer, une image virtuelle de la situation dentaire,
ii) puis on construit sur ordinateur, d'après cette image, un modèle de pièce de restauration dentaire,
iii) puis on fait polymériser couche par couche, par irradiation lumineuse sélective, un matériau de dentisterie conforme à l'une des revendications 1 à 11, pour former la restauration dentaire,
iv) et l'on nettoie ensuite la pièce brute, éventuellement en la traitant avec un solvant,
v) puis le cas échéant, on fait durcir davantage cette pièce brute, en l'irradiant de lumière et/ou en la chauffant à une température supérieure à 50 °C.

**13.** Utilisation d'un matériau conforme à l'une des revendications 1 à 11 en tant que matériau de dentisterie ou pour la fabrication ou la réparation de pièces façonnées de dentisterie.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 10562995 B2
- US 10568814 B2
- EP 3020361 A1
- EP 3494954 A1
- EP 3564206 A1
- WO 2014078537 A1
- US 20180000570 A1
- US 10299896 B2
- US 20190053883 A1
- EP 3564282 A1
- EP 2492289 A1


### In der Beschreibung aufgeführte Nicht-Patentliteratur


- **J. VIOHL** ; **K. DERMANN** ; **D. QUAST** ; **S. VENZ**. Die Chemie zahnärztlicher Füllungskunststoffe. Carl Hanser Verlag, 1986, 21-27
- **A. PEUTZFELDT**. Resin composites in dentistry: The monomer systems. *Eur. J. Oral. Sci.*, 1997, vol. 105, 97-116
- **N. MOSZNER** ; **T. HIRT**. New Polymer-Chemical Developments in Clinical Dental Polymer Materials: Enamel-Dentin Adhesives and Restorative Composites. *J. Polym. Sci. Part A: Polym. Chem.*, 2012, vol. 50, 4369-4402